# EUROPEAN PATENT SPECIFICATION

(11) **EP 3 788 977 B1**
(45) Date of publication and mention of the grant of the patent: **14.06.2023**
(21) Application number: 20188365.9
(22) Date of filing: 29.07.2020
(51) Int. Cl.: A61B 34/00, A61B 1/00, A61B 18/14, A61B 34/37, A61B 90/00, A61B 17/00, A61B 18/00, A61B 34/20, A61B 34/30

(54) **SURGICAL SYSTEM**
CHIRURGISCHES SYSTEM
SYSTÈME CHIRURGICAL

(30) Priority: 29.07.2019 JP 2019139176
(43) Date of publication of application: 10.03.2021
(73) Proprietor: Medicaroid Corporation, Kobe-shi, Hyogo 650-0047 (JP)
(72) Inventor: Kazuki, Ishihara, Kobe-shi Hyogo, 650-0047 (JP)
(74) Representative: Hoffmann Eitle

(56) References cited:
- WO-A1-2018/013197
- WO-A1-2018/170031
- US-B2- 8 862 268
- US-B2- 9 301 811

## Description

### BACKGROUND

The disclosure may relate to a surgical system, and may particularly relate to a surgical system including a remote control apparatus for operating surgical instruments.

In a related art, there has been known a surgical system including a remote control apparatus for operating surgical instruments (e.g., see Documents 1 and 2).

Documents 1 and 2 disclose a surgical system that includes: manipulators that support an endoscope for capturing an image of a surgery site and surgical instruments; a display device that displays the image captured by the endoscope; and a remote control apparatus that includes an input device to remotely operate the surgical instruments. The surgical system disclosed in Document 1 is configured, to display, at an edge portion of the display area of the display device, a symbol to indicate a position of the surgical instrument that is located out of the field of view of the endoscope. The surgical system disclosed in Document 2 is configured to display, at an edge portion of a display area of the display device, an icon that indicates information of a surgical instrument operated by the right hand, an icon that indicates information of a surgical instrument operated by the left hand, an icon that indicates information of a surgical instrument for replacement, an energy ball icon, and multiple master control icons.
Patent Document 1: JP 2013-188574 A
Patent Document 2: US 8,418,073 B2
Document US 8,862,268 B2 describes a user interface controller for a teleoperated surgical system and a surgical system.

### SUMMARY

As described above, the surgical system disclosed in Document 1 displays, at the edge portion of the display area of the display device, the symbol that indicates the position of the surgical instrument that is out of the field of view of the endoscope. Accordingly, if the display device disclosed in Patent Document 2 displays a symbol such as being disclosed in Patent Document 1, the symbol may be overlapped with the various information displayed at the edge portion of the display area of the display device and thus be difficult to be see. Further, it may be desired to provide a display device in which positional relationships of a plurality of surgical instruments with respect to the field of view of the endoscope can be more easily grasped than the display device of Patent Document 1.

An object of the present disclosure is to provide a control apparatus, a surgical system and a remote control apparatus therefor or thereof that are configured for and capable of displaying information of positional relationships of a plurality of surgical instruments with respect to a field of view of an endoscope without being overlapped with other information. Another object of one or more examples of the disclosure may be to provide a surgical system with respective components thereof in which positional relationships of a plurality of surgical instruments with respect to a field of view of an endoscope can be easily grasped. This greatly assists the operator (surgeon) of the surgical system to more safely, precisely and efficiently operate and control the medical equipment of the surgical system and to thus improve the medical treatment quality, safety, efficiency and effectiveness.

The surgical system of the present invention is defined by appended independent claim 1. The dependent claims are related to optional features and distinct embodiments. All embodiments and aspects described herein below not falling under the ambit of the claimed surgical system are not part of the claimed invention.

An aspect of the present disclosure is a control apparatus, optionally and non-limiting a computer-based display controller, for a remote control apparatus for a surgical system having a patient-side apparatus, wherein said control apparatus is configured and arranged to control at least a display device of the remote control apparatus, and further configured and arranged to display, on the display device, a graphical user interface overlapped with the image captured by an endoscope of the patient-side apparatus, the graphical user interface including a first area that indicates information on/of a first surgical instrument of the patient-side apparatus (200), a second area that indicates information on/of a third surgical instrument of the patient-side apparatus, a third area that indicates information on/of a second surgical instrument patient-side apparatus, which are arranged in one of an upper end portion and a lower end portion in a display area of the display device and further including, at a position different from that of the one of the upper end portion and the lower end portion in the display area, a surgical instrument position display area that indicates positional relationships of the first, second, third surgical instruments with respect to a field of view of the endoscope.

The mentioned remote control apparatus, surgical system and patient-side apparatus are optionally those to be detailed below in any of their conceivable configurations. Preferably, all the mentioned components are interrelated, form sub-components and systems, work together and complement each other. It is particularly preferred as part of this invention to have a surgical system that includes the remote control apparatus and the patient-side apparatus as components thereof. The control apparatus in turn is a component of the remote control apparatus or a separate component of the surgical system in addition to said remote control apparatus and patient-side apparatus, as will be detailed below.

A further aspect of the present disclosure is a surgical system that includes: a patient-side apparatus with operable medical equipment, for example, a first manipulator that supports a first surgical instrument; a second manipulator that supports a second surgical instrument; a third manipulator that supports a third surgical instrument; a fourth manipulator that supports an endoscope configured to capture an image of a surgery site; and a remote control apparatus for operating and controlling the patient-side apparatus that includes a display device configured to display the image captured by the endoscope, a first operation handle for right hand to operate the first surgical instrument, and a second operation handle for left hand to operate the second surgical instrument; and a control apparatus that controls and is configured to control the display device. The control apparatus displays and is configured to display, on the display device, a graphical user interface overlapped with the image captured by the endoscope, the graphical user interface including a first area that indicates information on the first surgical instrument, a second area that indicates information on the third surgical instrument, a third area that indicates information on the second surgical instrument, which are arranged in one of an upper end portion and an lower end portion in a display area of the display device and further including, at a position different from that of the one of the upper end portion and the lower end portion in the display area, a surgical instrument position display area that indicates positional relationships of the first, second, third surgical instruments with respect to a field of view of the endoscope.

An aspect of the present disclosure and not part of the claimed invention is a remote control apparatus for or of a surgical system, preferably the one detailed above and below, that includes: a display device configured to display an image captured by an endoscope, a first operation handle for right hand to operate a first surgical instrument, and a second operation handle for left hand to operate a second surgical instrument; and a control apparatus that controls and is configured to control the display device. The control apparatus displays and is configured to display, on the display device, a graphical user interface overlapped with the image captured by the endoscope, the graphical user interface including a surgical instrument position display area that indicates positional relationships of the first, second surgical instruments and a third surgical instrument for replacement with respect to a field of view of the endoscope. The surgical instrument position display area includes an in-field portion corresponding to an inside of the field of view of the endoscope and an out-of-field portion corresponding to an outside of the field of view of the endoscope around the field of view. The out-of-field portion is configured, when one or more of the first, second, third surgical instruments that are located outside the field of view of the endoscope, to display information of each of one or more of the first, second, third surgical instruments that are located outside the field of view of the endoscope.

An aspect of the present disclosure and not part of the claimed invention is a method of or for operating and/or controlling a surgical system, preferably the one detailed above and below and or with a method of or for displaying information in a remote control apparatus. The remote control apparatus, which may preferably be the one detailed above and below, may include: a display device configured to display an image captured by an endoscope; a first operation handle for right hand to operate a first surgical instrument; and a second operation handle for left hand to operate a second surgical instrument. The method includes: displaying, on the display device, a graphical user interface overlapped with the image captured by the endoscope, the graphical user interface including a surgical instrument position display area that indicates positional relationships of the first, second surgical instruments and a third surgical instrument for replacement with respect to a field of view of the endoscope; and displaying, when the first and second instruments are located in the field of view of the endoscope and the third instrument is located outside the field of view of the endoscope, information of the first and second surgical instruments in an in-field portion in the surgical instrument position display area and information of the third surgical instrument in an out-of-field portion in the surgical instrument position display area. An optional further step is then the operation and control of the medical equipment of the patient-side apparatus via the remote control apparatus based on the information provided and conditioned by the display device and a respective control apparatus.

### BRIEF DESCRIPTION OF DRAWINGS

FIG. 1 is a diagram illustrating an overview of a surgical system according to the invention;
FIG. 2 is a block diagram illustrating a view of a control-related configuration of the surgical system;
FIG. 3 is a diagram illustrating a perspective view of an operation pedal section of a remote control apparatus;
FIG. 4 is a diagram illustrating views for explaining assignment examples of the operation pedal section of the remote control apparatus;
FIG. 5 is a diagram illustrating a view of an example of arrangements of surgical instruments;
FIG. 6 is a diagram illustrating a view of an example of a screen displayed on a display device according to the invention;
FIG. 7 is a diagram illustrating a view of an example of a screen of the display device in which a surgical instrument position display area is displayed;
FIG. 8 is a diagram illustrating a view of an example of a screen of the display device in which a surgical instrument position display area is hidden (not displayed);
FIG. 9 is a diagram illustrating a view of an example of a screen of the display device when a switch pedal is operated;
FIG. 10 is a diagram illustrating a view of an example of a screen of the display device when a foot is at an operation preparation position for a clutch pedal;
FIG. 11 is a diagram illustrating a view of an example of a screen of the display device when the clutch pedal is operated;
FIG. 12 is a diagram illustrating a view of an example of a screen of the display device when a clutch switch (right) is operated;
FIG. 13 is a diagram illustrating a view of an example of a screen of the display device when a clutch switch (left) is operated;
FIG. 14 is a diagram illustrating a view of an example of a screen of the display device when a foot is at an operation preparation position for a camera pedal;
FIG. 15 is a diagram illustrating a view of an example of a screen of the display device when the camera pedal is operated;
FIG. 16 is a diagram illustrating a view of an example of a screen of the display device when a foot is at an operation preparation position for a cutting pedal (right);
FIG. 17 is a diagram illustrating a view of an example of a screen of the display device when the cutting pedal (right) is operated;
FIG. 18 is a diagram illustrating a view of an example of a screen of the display device when a foot is at an operation preparation position for a coagulation pedal (right);
FIG. 19 is a diagram illustrating a view of an example of a screen of the display device when the coagulation pedal (right) is operated;
FIG. 20 is a diagram illustrating a view of an example of a screen of the display device when feet are at operation preparation positions for a cutting pedal (left) and the clutch pedal;
FIG. 21 is a diagram illustrating a view of an example of a screen of the display device on which a function limit notification is displayed; and
FIG. 22 is a diagram illustrating a view of an example of a screen of the display device on which an error notification is displayed.

### DETAILED DESCRIPTION

Descriptions are provided hereinbelow for one or more embodiments based on the drawings. In the respective drawings referenced herein, the same constituents are designated by the same reference numerals and duplicate explanation concerning the same constituents is omitted. All of the drawings are provided to illustrate the respective examples only and are not meant to limit the present invention in any way.

### (Configuration of Surgical System)

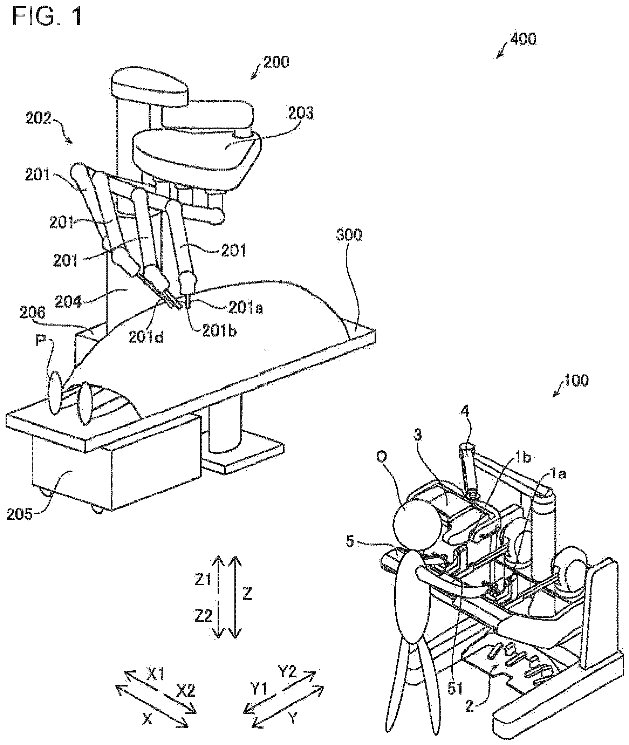

A configuration of a surgical system 400 is described with reference to FIGS. 1 to 22, wherein only the surgical system according to and with the components defined in the appended claims is part of the claimed invention.

As illustrated in FIG. 1, the surgical system 400 includes a remote control apparatus 100 and a patient-side apparatus 200. The remote control apparatus 100 is provided for teleoperation of medical equipment included in the patient-side apparatus 200. When an operator O, as a surgeon, inputs an action mode instruction to be executed by the patient-side apparatus 200 to the remote control apparatus 100, the remote control apparatus 100 transmits the action mode instruction to the patient-side apparatus 200 through a controller 206. In response to the action mode instruction transmitted from the remote control apparatus 100, the patient-side apparatus 200 operates the medical equipment, such as surgical instruments 201a to 201c and an endoscope 201d, attached to manipulators 201. This allows for minimally invasive surgery.

The patient-side apparatus 200 constitutes an interface to perform a surgery for a patient P. The patient-side apparatus 200 optionally has the form or configuration of a highly maneuverable robot arm. The patient-side apparatus 200 is placed beside an operation table 300 on which the patient P lies, preferably such that the operation table 300 and the patient P are within operational reach and distance of the medical devices and equipment of the patient-side apparatus 200. The patient-side apparatus 200 includes the manipulators 201, preferably in the form of elongated arms. One of the manipulators 201 supports the endoscope 201d while the others hold the surgical instruments 201a to 201c. The manipulators 201 are commonly supported by a platform 203. Each of the manipulators 201 includes joints. Each joint includes a driver including a servo-motor and a position detector such as an encoder. The manipulators 201 are configured so that the medical equipment attached to each manipulator 201 is controlled by a driving signal given through the controller 206, to perform a desired movement.

The platform 203 is supported by a positioner 202 placed on the floor of an operation room. The positioner 202 includes a column 204 and a base 205. The column 204 includes an elevating shaft adjustable in the vertical direction. The base 205 includes wheels and is movable on the floor surface.

The surgical instruments 201a to 201c as the medical equipment are detachably attached to the distal ends of three of the four manipulators 201, respectively. The surgical instruments 201a to 201c each include a housing, an elongate shaft, and an end effector. The housing is attached to the corresponding one of the manipulators 201. The end effector is provided at a distal end of the shaft. The end effector is grasping forceps, scissors, a hook, a high-frequency knife, a snare wire, a clamp, or a stapler, for example. The end effector is not limited to those and can be various types of treatment tools. In surgeries using the patient-side apparatus 200, the three manipulators 201 introduce the surgical instruments 201a to 201c into the body of the patient P through a cannula (trocar) placed on the body surface of the patient P. The end effector of each of the surgical instruments 201a to 201c is then located near the surgery site. The surgical instrument 201a, the surgical instrument 201b, and the surgical instrument 201c are examples of a "first surgical instrument", a "second surgical instrument", and a "third surgical instrument", respectively.

To the distal end of one of the four manipulators 201, the endoscope 201d as the medical equipment is detachably attached. The endoscope 201d captures an image within the body cavity of the patient P. The captured image is outputted to the remote control apparatus 100. The endoscope 201d may be a 3D endoscope capable of capturing a three-dimensional image or a 2D endoscope. In surgeries using the patient-side apparatus 200, the one of the manipulators 201 introduces the endoscope 201d into the body of the patient P through a trocar placed on the body surface of the patient P. The endoscope 201d is then located near the surgery site.

The remote control apparatus 100 constitutes the interface with the operator O. The remote control apparatus 100 is an apparatus that allows the operator O to operate the medical equipment held by the manipulators 201. Specifically, the remote control apparatus 100 is configured to transmit action mode instructions which are inputted by the operator O and are to be executed by the surgical instruments 201a to 201c and endoscope 201d, to the patient-side apparatus 200 through the controller 206. The remote control apparatus 100 is installed beside the operation table 300 so that the operator O can see the state of the patient P very well while operating the remote control apparatus 100, for example. The remote control apparatus 100 may be configured to transmit the action mode instructions wirelessly and installed in a room different from the operation room where the operation table 300 is installed.

The action modes to be executed by the surgical instruments 201a to 201c include a mode of actions to be taken by each of the surgical instruments 201a to 201c (a series of positions and postures) and actions to be executed by the function of each of the surgical instruments 201a to 201c. For one of the surgical instruments 201a to 201c which is a pair of grasping forceps, for example, the action mode to be executed by the surgical instruments 201a to 201c includes setting of roll and pitch positions of the wrist of the end effector and the action to open or close the jaws. For one of the surgical instruments 201a to 201c which is a high-frequency knife, the action mode to be executed by the surgical instruments 201a to 201c includes vibration of the high-frequency knife, specifically, supply of current to the high-frequency knife. For one of the surgical instruments 201a to 201c which is a snare wire, the action mode to be executed by the surgical instruments 201a to 201c includes a capturing action and an action to release the captured object and moreover includes an action to supply current to a bipolar or monopolar instrument to burn off the surgery site.

The action mode to be executed by the endoscope 201d includes setting of the position and posture of the tip of the endoscope 201d or setting of the zoom magnification, for example.

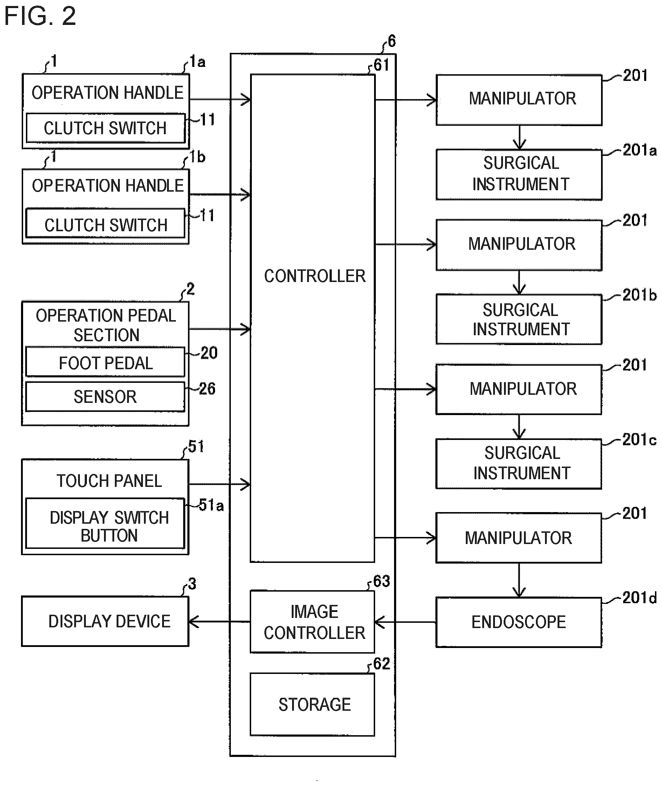

As illustrated in FIGS. 1 and 2, the remote control apparatus 100 includes operation handles 1a and 1b, an operation pedal section 2, a display device 3, a display device supporting arm 4 supporting the display device 3, an armrest 5 to support the arms of the operator O, and a control apparatus 6. Optionally, the display device 3 may be or may comprise a separate/external structure/entity, for example a rack-structure, with extra monitors that is not an integral part of the assembly of the other components of/forming the remote control apparatus but can be placed next to it and is electrically connected to it. Optionally, the control apparatus 6 is a separate/external structure/entity and not integral part of an assembly of the other components of/forming the remote control apparatus and may be included in the separate/external structure/entity of the display device. Optionally, the control apparatus is a separate, stand-alone controller or PC-system as a possible part/component of the overall surgical system 400.

The operation handles 1a and 1b are provided in order to remotely operate the medical equipment held by the manipulators 201. Specifically, the operation handles 1a and 1b accept operations by the operator O for operating the medical equipment (the surgical instruments 201a to 201c and the endoscope 201d). The operation handles 1a and 1b are arranged side by side in the X direction. The operation handle 1a is arranged on the right side (the X2 side) to be operated by the right hand of the operator O. The operation handle 1b is arranged on the left side (the X1 side) to be operated by the left hand of the operator O. The operation handle 1a and the operation handle 1b are examples of a "first operation handle" and a "second operation handle", respectively.

The operation handles 1a and 1b are arranged to extend from the rear side (the Y2 side) of the remote control apparatus 100 to the front side (the Y1 side) thereof. The operation handles 1a and 1b are configured to be movable within a predetermined three-dimensional operation region. Specifically, the operation handles 1a and 1b are configured to be movable in the up-down direction (the Z direction), the right-left direction (the X direction), and the front-rear direction (the Y direction).

The right hand-operation handle 1a is provided to remotely operate the corresponding surgical instrument 201a. The left hand-operation handle 1b is provided to remotely operate the corresponding surgical instrument 201b. As illustrated in FIG. 2, a clutch switch 11 is provided in each of the operation handles 1a and 1b. The clutch switch 11 is operated when temporarily disconnecting the control-related connections between the manipulators 201 and the operation handles 1a and 1b. When the clutch switch 11 of the operation handle 1a is operated, the control-related connection between the operation handle 1a and one of the manipulators 201 to which the surgical instrument 201a is provided is temporarily disconnected. When the clutch switch 11 of the operation handle 1b is operated, the control-related connection between the operation handle 1b and another one of the manipulators 201 to which the surgical instrument 201b is provided is temporarily disconnected.

The remote control apparatus 100 and patient-side apparatus 200 constitute a master-slave system in terms of controlling motion of the manipulators 201. Specifically, the operation handles 1a and 1b constitute an operating section on the master side in the master-slave system, and the manipulators 201 supporting the medical equipment constitute an operating section on the slave side. When the operator O operates the operation handles 1a and 1b, the motion of the manipulators 201 is controlled so that the distal ends (the end effectors of the surgical instruments 201a to 201c or the endoscope 201d) of the manipulators 201 move following the movement of the operation handles 1a and 1b.

The patient-side apparatus 200 is configured to control the motion of the manipulators 201 in accordance with the set motion scaling ratio. When the motion scaling ratio is set to 1/2, for example, the end effectors of the surgical instruments 201a to 201c move 1/2 of the movement distance of the operation handles 1a and 1b. This allows for precise fine surgery.

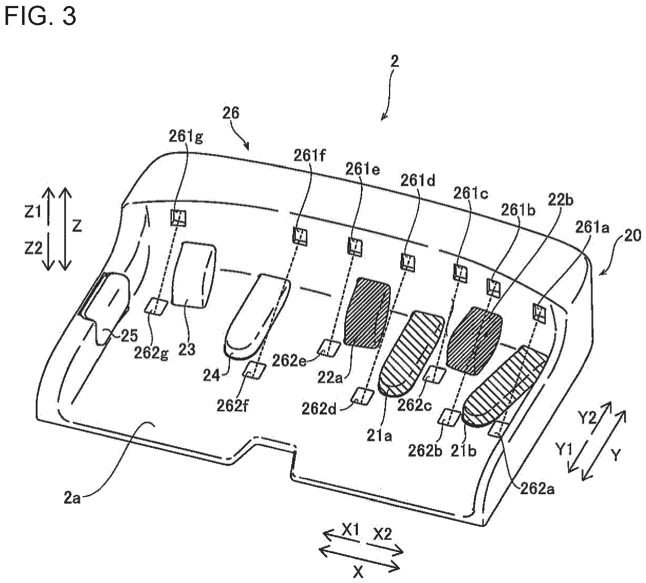

As illustrated in FIG. 3, the operation pedal section 2 includes foot pedals 20 that execute functions of the medical equipment. The foot pedals 20 are arranged on a base 2a. The foot pedals 20 include coagulation pedals 21a and 21b, cutting pedals 22a and 22b, a clutch pedal 23, and a camera pedal 24. Additionally, the operation pedal section 2 is provided with a switch pedal 25. The coagulation pedals 21a and 21b, cutting pedals 22a and 22b, clutch pedal 23, and camera pedal 24 are operated by being pressed downward. The switch pedal 25 is operated by being pressed in the horizontal direction. The foot pedals 20 are operated by the foot of the operator O. Note that the coagulation pedal 21b and the cutting pedal 22b are examples of a "first foot pedal". The coagulation pedal 21a and the cutting pedal 22a are examples of a "second foot pedal". The camera pedal 24 is an example of a "third foot pedal". The clutch pedal 23 is an example of a "fourth foot pedal". The switch pedal 25 is an example of a "fifth foot pedal".

The operation pedal section 2 includes sensors 26 that detect the presence of the foot operating the foot pedals 20. The sensors 26 include light emitters 261a, 261b, 261c, 261d, 261e, 261f, and 261g and light receivers 262a, 262b, 262c, 262d, 262e, 262f, and 262g. The sensors 26 detect the presence of the foot when the light from the emitter 261a, 261b, 261c, 261d, 261e, 261f, and 261g is blocked by the foot and the light reception by the receiver 262a, 262b, 262c, 262d, 262e, 262f, and 262g is interrupted. Specifically, the sensors 26 are blockage type sensors. The sensors 26 are provided to detect that the foot of the operator O is put on any one of the foot pedals 20. In other words, the sensors 26 detect setting of the foot of the operator O at an operation preparation position of the foot pedal 20.

The detection information detected by the sensors 26 is transmitted to a controller 61, and the controller 61 that receives the detection information determines whether there is the foot operating the corresponding foot pedals 20.

The coagulation pedals 21a and 21b enable surgical instruments to coagulate surgery sites. Specifically, when the coagulation pedal 21a or 21b is operated, voltage for coagulation is applied to corresponding one of the surgical instruments 201a, 201b and 201c to coagulate surgery sites. The coagulation pedal 21a is located to the left (on the X1 side) of the coagulation pedal 21b. The coagulation pedal 21a is used in relation to the surgical instrument 201b controlled by the left hand-operation handle 1b. The coagulation pedal 21b is used in relation to the surgical instrument 201a controlled by the right hand-operation handle 1a. The coagulation pedals 21a and 21b are in a first color. For example, the coagulation pedals 21a and 21b are in blue as the first color.

The cutting pedals 22a and 22b enable the surgical instruments to cut surgery sites. Specifically, when the cutting pedal 22a or 22b is operated, voltage for cutting is applied to corresponding one of the surgical instruments 201a, 201b, and 201c to cut surgery sites. The cutting pedal 22a is located to the left (on the X1 side) of the cutting pedal 22b. The cutting pedal 22a is used in relation to the surgical instrument 201b controlled by the left hand-operation handle 1b. The cutting pedal 22b is used in relation to the surgical instrument 201a controlled by the right hand-operation handle 1a. The cutting pedals 22a and 22b are in a second color different from the first color. For example, the cutting pedals 22a and 22b are in yellow as the second color.

Specifically, the coagulation pedal 21b and the cutting pedal 22b execute the function of the surgical instrument 201a operated by the right hand-operation handle 1a. The coagulation pedal 21a and the cutting pedal 22a execute the function of the surgical instrument 201b operated by the left hand-operation handle 1b.

The clutch pedal 23 is operated to execute a clutch function. Specifically, the clutch pedal 23 is used when temporarily disconnecting the control-related connections between the manipulators 201 and the operation handles 1a and 1b to stop the operation of the surgical instruments. During the operation of the clutch pedal 23, the manipulators 201 of the patient-side apparatus 200 does not move even when the operation handles 1a and 1b are operated. For example, when the operation handle 1a (1b) come close to the end of the movable region during the operation, it is possible to move the operation handle 1a (1b) back to near the central position by operating the clutch pedal 23 and temporarily disconnecting the control-related connections. Then, once the operation of the clutch pedal 23 is quitted, the manipulators 201 and the operation handles 1a and 1b are connected again, and operation of the operation handles 1a and 1b can be restarted from near the central position. In this case, the clutch pedal 23 is operated to temporarily disconnect the whole control-related connections between both the operation handles 1a and 1b and the manipulators 201. On the other hand, the clutch switch 11 provided in each of the operation handles 1a and 1b is operated to temporarily disconnect the control-related connections between either of the operation handles 1a and 1b and corresponding one of the manipulators 201, individually.

The camera pedal 24 is used to control the position and orientation of the endoscope 201d that captures images within the body cavity. That is, the camera pedal 24 executes a function of the endoscope 201d. Specifically, the camera pedal 24 enables control of the endoscope 201d by the operation handles 1a and 1b. That is, the position and orientation of the endoscope 201d are controllable by the operation handles 1a and 1b while the camera pedal 24 is being pressed. The endoscope 201d is controlled by using both of the right and left operation handles 1a and 1b, for example. Specifically, when the operator O rotates the right and left operation handles 1a and 1b about the middle point between the right and left operation handles 1a and 1b, the endoscope 201d is rotated. When the operator O presses the right and left operation handles 1a and 1b together, the endoscope 201d goes further into the body cavity. When the operator O pulls the right and left operation handles 1a and 1b together, the endoscope 201d retracts. When the operator O moves the right and left operation handles 1a and 1b together up, down, right, and left, the endoscope 201d moves up, down, right, and left, respectively.

The switch pedal 25 is used to switch the manipulators 201 to be operated by the operation handles 1a and 1b. For example, four manipulators 201 are provided, and within three of the manipulators 201 respectively holding the surgical instruments 201a to 201c, the manipulators 201 operated by the right and left operation handles 1a and 1b are switched by operating the switch pedal 25. Specifically, the switch pedal 25 is operated to replace the surgical instrument 201a operated by the right hand-operation handle 1a or the surgical instrument 201b operated by the left hand-operation handle 1b with the surgical instrument 201c, which is provided to change the type of the operation. The switch pedal 25 is operated by being pressed leftward (in the X1 direction). For example, the manipulator 201 operated by the right hand-operation handle 1a is switched by operating the switch pedal 25. In this case, the manipulator 201 operated by the right hand-operation handle 1a is switched without changing the manipulator 201 operated by the left hand-operation handle 1b. For example, the manipulator 201 operated by the left hand-operation handle 1b is switched by operating the switch pedal 25. In this case, the manipulator 201 operated by the left hand-operation handle 1b is switched without changing the manipulator 201 operated by the right hand-operation handle 1a.

As illustrated in FIG. 3, the foot pedals 20 are arranged from the left side (the X1 side) to the right side (the X2 side) in order of the switch pedal 25, the clutch pedal 23, the camera pedal 24, the cutting pedal 22a, the coagulation pedal 21a, the cutting pedal 22b, and the coagulation pedal 21a.

The arrangement of the foot pedals 20 illustrated in FIG. 3 is suitable for operating the pairs of coagulation pedals 21a and 21b and cutting pedals 22a and 22b assigned to the respective right and left operation handles 1a and 1b with only the right foot. The pedals arrangement may be as follows: the pair of coagulation pedal 21a and cutting pedal 22a is located to the left (the X1 side) of the clutch pedal 23 and camera pedal 24, and the clutch pedal 23 and camera pedal 24 are located at the center (between the pair of coagulation pedal 21a and cutting pedal 22a and the pair of coagulation pedal 21b and cutting pedal 22b). This arrangement is suitable for operating the coagulation pedal 21a and cutting pedal 22a assigned to the left hand-operation handle 1b with the left foot while operating the coagulation pedal 21b and cutting pedal 22b assigned to the right hand-operation handle 1a with the right foot.

The base 2a on which the foot pedals 20 are arranged is movable in the horizontal direction. Specifically, two sides in the axial direction (the X direction) of the base 2a are connected with a base of the remote control apparatus 100 with sliding bearings, and the operation pedal section 2 is slidable and movable in the depth direction (the front-rear direction, the Y direction). The operation pedal section 2 can be moved in the depth direction electrically by a driver device such as a motor provided in the base of the remote control apparatus 100. This makes it possible to adjust the positions of the foot pedals 20 depending on the operation posture, physique, or preference of the operator O.

The sensors 26 detects whether there is the foot that is operating one of the foot pedals 20. The emitters 261a and 261b and the receivers 262a and 262b detect the foot coming closer to the coagulation pedal 21b. Specifically, when both the receivers 262a and 262b detect the foot, the controller 61 determines that the foot pedal 20 to be operated is the coagulation pedal 21b. The emitters 261and 261c and the receivers 262b and 262c detect the foot coming closer to the cutting pedal 22b. Specifically, when both the receivers 262b and 262c detect the foot, the controller 61 determines that the foot pedal 20 to be operated is the cutting pedal 22b.

The emitters 261c and 261d and the receivers 262c and 262d detect the foot coming closer to the coagulation pedal 21a. Specifically, when both the receivers 262c and 262d detect the foot, the controller 61 determines that the foot pedal 20 to be operated is the coagulation pedal 21a. The emitters 261d and 261e and the receivers 262d and 262e detect the foot coming closer to the cutting pedal 22a. Specifically, when both the receivers 262d and 262e detect the foot, the controller 61 determines that the foot pedal 20 to be operated is the cutting pedal 22a.

The emitter 261f and the receiver 262f detects the foot coming closer to the camera pedal 24. Specifically, when the receiver 262f detects the foot, the controller 61 determines that the foot pedal 20 to be operated is the camera pedal 24. The emitter 261g and the receiver 262g detects the foot coming closer to the clutch pedal 23. Specifically, when the receiver 262g detects the foot, the controller 61 determines that the foot pedal 20 to be operated is the clutch pedal 23.

The emitters 261a to 261g include light-emitting elements such LEDs. The emitters 261a to 261g are configured to emit visible light or invisible light such as infrared light. The receivers 262a to 262g include light-receiving elements. Each pair of emitters 261a to 261g and receivers 262a to 262g is arranged along the Y direction in the plan view. Specifically, as illustrated as a broken line in FIG. 3, the light from each of the emitters 261a to 261g is emitted substantially along the Y direction in the plan view.

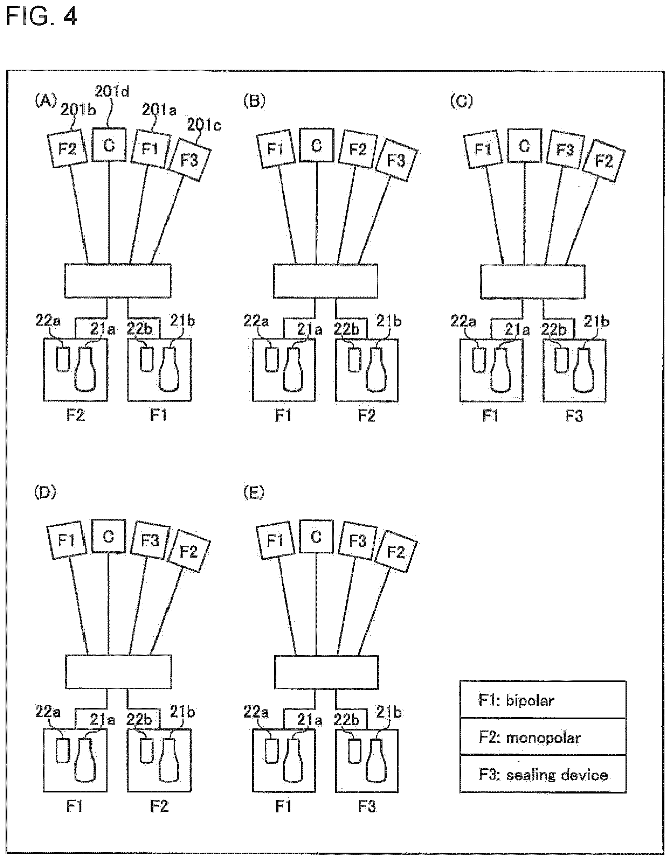

An assignment example of the coagulation pedals 21 (21a and 21b) and the cutting pedals 22 (22a and 22b) on the operation pedal section 2 is described with reference to FIG. 4. The coagulation pedal 21a and the cutting pedal 22a are used as a pair, while the coagulation pedal 21b and the cutting pedal 22b are used as a pair. In this case, it is possible to use a pair of forceps (e.g., a grasper) to cut and coagulate the surgery site. When a pair of forceps is used for cutting and coagulating, high voltage is applied for cutting, and voltage lower than the cutting case is applied for coagulating. Specifically, it is possible to coagulate and cut the surgery site by selecting and using the coagulation pedal 21a (21b) and the cutting pedal 22a (22b). Although a grasper and the like can be used for cutting and coagulating, a sealing device for coagulating may also be used as a dedicated tool or used concurrently with another tool. This is because the sealing device often has an additional function such as automatic termination of power supply that is executed when coagulating is completed.

In the example illustrated in FIG. 4, a pair of bipolar forceps F1, a pair of monopolar forceps F2, and a sealing device F3 as the medical equipment and the endoscope 201d are attached to the four manipulators 201. The positional relationships between the four manipulators 201 are recognized by the position detector provided for each manipulator. The positional relationships between the manipulators in the right-left direction are determined based on the positions thereof seen from the platform 203. In FIG. 4A, the pair of monopolar forceps F2 is located to the left of one of the manipulators 201 to which the endoscope 201d is attached, and the pair of bipolar forceps F1 and the sealing device F3 are located to the right of the manipulator 201 to which the endoscope 201d is attached in this order from left. In the assignment of the coagulation pedals 21a and 21b and cutting pedals 22a and 22b, the manipulator 201 to which the surgical instrument 201b is attached is assigned to the left-side foot pedals (the coagulation pedal 21a and the cutting pedal 22a), and the manipulator 201 to which the surgical instrument 201a, which is a surgical instrument to the right of the manipulator 201 to which the surgical instrument 201b is attached, is assigned to right-side foot pedals (the coagulation pedal 21b and cutting pedal 22b). Specifically, in FIG. 4A, the pair of monopolar forceps F2 is assigned to the left-side foot pedals (the coagulation pedal 21a and the cutting pedal 22a), while the pair of bipolar forceps F1 is assigned to the right-side foot pedals (the coagulation pedal 21b and the cutting pedal 22b). When only two surgical instruments are attached, the manipulators 201 are assigned to the left-side foot pedals and the right-side foot pedals in order from the one on the leftmost side of the manipulator 201 to which the endoscope 201d is attached. When only one surgical instrument is attached, the manipulator 201 is assigned to the left-side foot pedals.

In FIG. 4B, the pair of bipolar forceps F1 and the pair of monopolar forceps F2 are replaced with each other by an assistant (a nurse, for example). In this case, the types of the surgical instruments 201a and 201b are specified when the surgical instruments 201a and 201b are attached to the manipulators 201. For example, the IC of the interface may store information including model numbers of the instruments. The pair of bipolar forceps F1 is assigned to the left-side foot pedals (the coagulation pedal 21a and the cutting pedal 22a). The pair of monopolar forceps F2 is assigned to the right-side foot pedals (the coagulation pedal 21b and the cutting pedal 22b).

In FIG. 4C, the pair of monopolar forceps F2 and the sealing device F3 are replaced with each other by an assistant (a nurse, for example). In this case, the types of the surgical instruments 201a and 201b are specified when the surgical instruments 201a and 201b are attached to the manipulators 201. The pair of bipolar forceps F1 continues to be assigned to the left-side foot pedals (the coagulation pedal 21a and the cutting pedal 22a). The sealing device F3 is assigned to the right-side foot pedals (the coagulation pedal 21b and the cutting pedal 22b).

In FIG. 4D, the switch pedal 25 is operated to change which of the two manipulators 201 located on the right side is being activated. Specifically, the manipulators 201 to be controlled by the operation handle 1a is switched. The manipulator 201 to which the pair of bipolar forceps F1 is attached is controlled with the left hand-operation handle 1b while the manipulator 201 to which the pair of monopolar forceps F2 is attached is controlled with the right hand-operation handle 1a. The pair of bipolar forceps F1 continues to be assigned to the left-side foot pedals (the coagulation pedal 21a and the cutting pedal 22a). The pair of monopolar forceps F2 is assigned to the right-side foot pedals (the coagulation pedal 21b and the cutting pedal 22b).

In FIG. 4E, the switch pedal 25 is operated to change which of the two manipulators 201 on the right side is being activated. Specifically, the manipulators 201 to be controlled by the operation handle 1a are switched. The manipulator 201 to which the pair of bipolar forceps F1 is attached is controlled with the left hand-operation handle 1b while the manipulator 201 to which the sealing device F3 is attached is controlled with the right hand-operation handle 1a. The pair of bipolar forceps F1 continues to be assigned to the left-side foot pedals (the coagulation pedal 21a and the cutting pedal 22a). The sealing device F3 is assigned to the right-side foot pedals (the coagulation pedal 21b and the cutting pedal 22b).

In some cases, a simple grasper which works without electricity is used when strong grip is mainly necessary. Such surgical instruments that cannot be supplied with current are not controlled with the coagulation pedals 21a and 21b and the cutting pedals 22a and 22b and are therefore not assigned to the coagulation pedals 21a (21b) and cutting pedals 22a (22b). The assignment of the coagulation pedals 21a and 21b and cutting pedals 22a and 22b is configured so that the manipulator 201 holding a surgical instrument that cannot be supplied with current is ignored.

The left-side foot pedals (the coagulation pedal 21a and the cutting pedal 22a) and right-side foot pedals (the coagulation pedal 21b and the cutting pedal 22b) may be assigned by another rule. For example, the manipulators 201 to the right and left of the manipulator 201 to which the endoscope 201d is attached may be always assigned to the foot pedals. For example, the one manipulator 201 to the left of the manipulator 201 to which the endoscope 201d is attached may be assigned to the left-side foot pedals (the coagulation pedal 21a and the cutting pedal 22a), while the left manipulator 201 among the two manipulators 201 located to the right of the manipulator 201 to which the endoscope 201d is attached is assigned to the right-side foot pedals (the coagulation pedal 21b and the cutting pedal 22b).

As illustrated in FIG. 1, the display device 3 displays an image captured by the endoscope 201d. The display device 3 includes a scope type display device or a non-scope type display device. In the example illustrated in FIG. 1, the display device 3 is the scope type display device. The scope type display device is a display unit that the operator O looks into, for example. The non-scope type display device is a concept including an open-type display unit that the operator O looks at without looking into and that has a flat screen, such as a normal personal computer display.

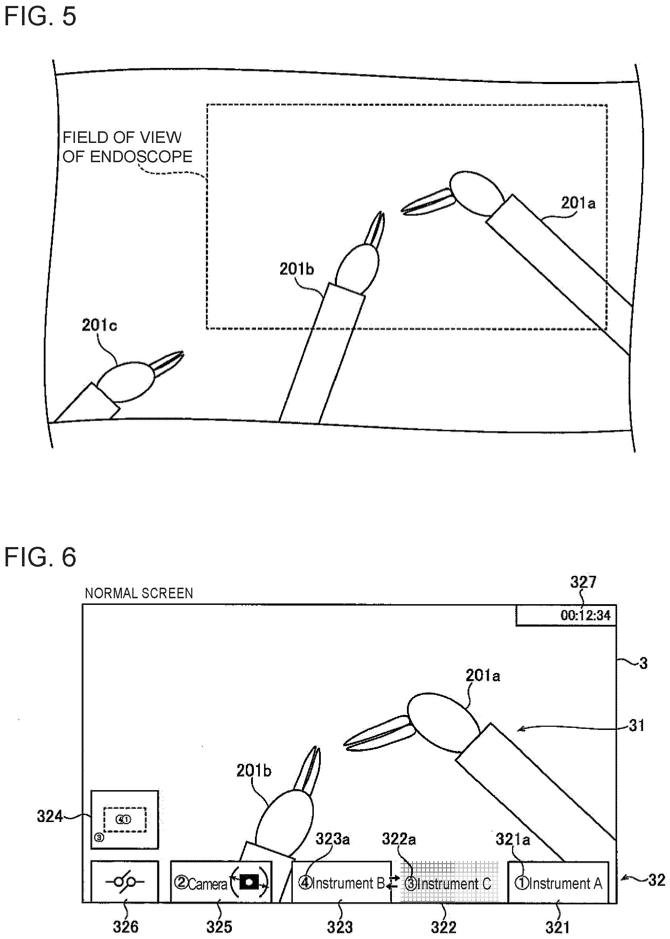

As illustrated in FIG. 1, the armrest 5 is provided with a touch panel 51. The touch panel 51 is configured to select various setting of the remote control apparatus 100. For example, the touch panel 51 is configured to receive an operation (a user input) to change a display form of a graphical user interface 32 (see FIG. 6) displayed on the display device 3.

As illustrated in FIG. 2, the control apparatus 6 such as a computer and optionally being a separate and/or external entity connected to but not physically integrated in the remote control apparatus, i.e., the assembly of its other components or being an entirely separate system, includes a controller 61, a storage 62, and an image controller 63, for example. The controller 61 includes a computing element such as a CPU. The storage 62 includes one or more memories, such as a ROM and a RAM. The control apparatus 6 is configured to control at least the display device 3, optionally but non-limiting, the control apparatus 6 is or is operating and functioning as a computer-based display controller The control apparatus 6 may be composed of a single controller performing centralized control or may be composed of plural controllers that perform decentralized control in cooperation with each other. The controller 61 determines whether the action mode instruction inputted by the operation handles 1a and 1b is to be executed by the surgical instruments 201a to 201c or to be executed by the endoscope 201d, depending on the state of the operation pedal section 2. The controller 61 transmits a signal to operate a corresponding one of the manipulators 201 based on the determination result and operations through the operation handles 1a and 1b.

The controller 61 receives the detection information on the presence of the foot of the operator O from the sensors 26 and determines whether there is the foot of the operator O. When it is determined that there is the foot of the operator O, the controller 61 controls the display device 3 to display the corresponding screen.

The storage 62 stores control programs corresponding to the types of the surgical instruments 201a to 201c, for example. The controller 61 reads the stored control programs according to the types of the attached surgical instruments 201a to 201c. The action mode instructions from the operation handles 1a and 1b and/or the operation pedal section 2 of the remote control apparatus 100 thereby causes the respective surgical instruments 201a to 201c to perform proper motions.

The image controller 63 transmits an image acquired by the endoscope 201d to the display device 3. The image controller 63 modifies the image if necessary.

As illustrated in FIG. 6, the control apparatus 6 is configured to display a graphical user interface 32 on the display device 3 while overlapping the graphical user interface 32 on an image 31 captured by the endoscope 201d. The graphical user interface 32 includes a first area 321 displaying information of the surgical instrument 201a operated by the right hand-operation handle 1a, a second area 322 displaying information of the surgical instrument 201c to change the type of the operation, and a third area 323 displaying information of the surgical instrument 201b, which are arranged in line side by side in this order from right to left.

According to the invention, the control apparatus 6 is configured to display the graphical user interface 32 on the display device 3 while overlapping the graphical user interface 32 on the image 31 captured by the endoscope 201d. The graphical user interface 32 includes the first area 321, the second area 322, and the third area 323 arranged in line, and also includes a surgical instrument position display area 324 which is provided at a position different from the line of the first, second, and third areas 321, 322, and 323, wherein the surgical instrument position display area 324 displays information indicating positional relationships of the surgical instruments 201a, 201b, and 201c with respect to a visual range (a field of view) of the endoscope 201d. Accordingly, the information indicating the positional relationships of the first, second, third surgical instruments 201a, 201b, and 201c with respect to the field of view of the endoscope 201d is displayed in the surgical instrument position display area 324. Therefore, the surgeon (the operator O) can intuitively grasp the positional relationships of the plurality of surgical instruments 201a, 201b, and 201c by viewing the surgical instrument position display area 324, and also can easily grasp the positional relationships of the plurality of surgical instruments 201a, 201b, and 201c with respect to the field of view of the endoscope 201d. Further, the surgical instrument position display area 324 that indicating the information on the positional relationships of the first, second, third surgical instruments 201a, 201b, and 201c with respect to the field of view of the endoscope 201d is displayed at the position different from the first, second, and third areas 321, 322, and 323 arranged in line. Therefore, it is possible to prevent the operator from having a difficulty to see the information by preventing the information from being overlapped.

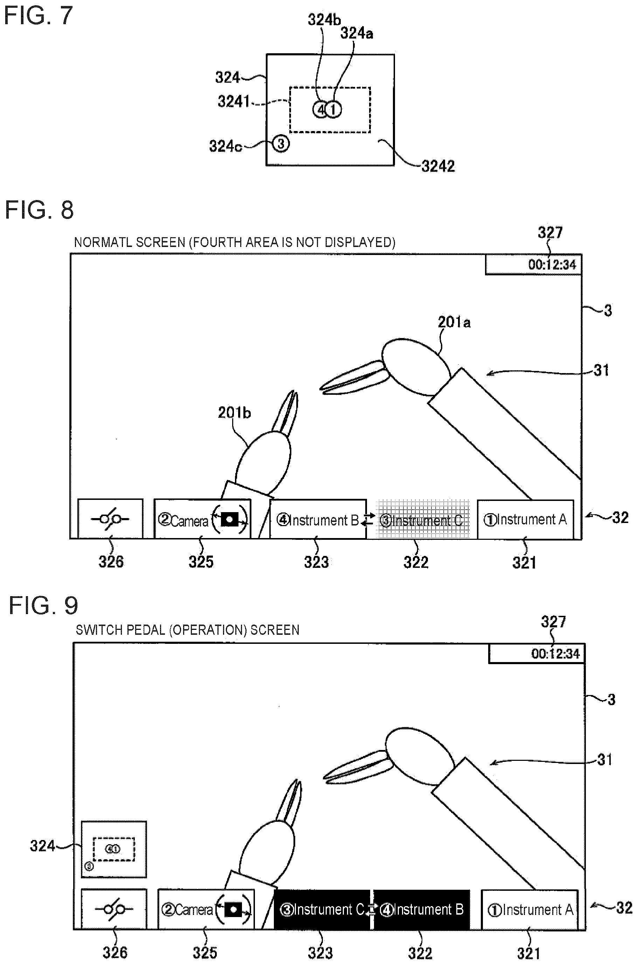

As illustrated in FIGS. 5 to 7, the information indicating each of the positions of the surgical instruments 201a, 201b, and 201c is displayed at a position in the surgical instrument position display area 324 corresponding to the position of each of the surgical instruments 201a, 201b, and 201c with respect to the field of view of the endoscope 201d. Specifically, the surgical instrument position display area 324 includes an in-field portion 3241 displaying information relating to the inside of the field of view of the endoscope 201d and an out-of-field portion 3242 displaying information relating to the outside of the field of view of the endoscope 201d. When the surgical instrument 201a, 201b or 201c is positioned in the field of view of the endoscope 201d, information of the surgical instrument 201a, 201b or 201c positioned in the field of view of the endoscope 201d is displayed at the corresponding position in the in-field portion 3241 of the surgical instrument position display area 324. Alternatively, when the surgical instrument 201a, 201b, or 201c is positioned outside the field of view of the endoscope 201d, information of the surgical instrument 201a, 201b or 201c positioned outside the field of view of the endoscope 201d is displayed at the corresponding position in the out-of-field portion 3242 of the surgical instrument position display area 324. Accordingly, even when one or more of the surgical instruments is outside the field of view of the endoscope 201d, it is possible to easily understand where the one or more surgical instruments outside the field of view of the endoscope 201d is located. Further, the positional relationships between the surgical instrument(s) outside the field of view of the endoscope 201d and the surgical instrument(s) within the field of view of the endoscope 201d can be easily grasped. In the example illustrated in FIG. 5, the surgical instruments 201a and 201b are located in the field of view of the endoscope 201d, and the surgical instrument 201c is located outside the field of view of the endoscope 201d. In this case, as illustrated in FIG. 7, in the in-field portion 3241 of the surgical instrument position display area 324, first identification information 324a of the surgical instrument 201a and third identification information 324b of the surgical instrument 201b are displayed. Further, in the out-of-field portion 3242 of the surgical instrument position display area 324, second identification information 324c of the surgical instrument 201c is displayed.

In the in-field portion 3241 of the surgical instrument position display area 324, the identification information of the surgical instruments 201a and 201b are displayed at positions corresponding to the positions of the distal ends of the surgical instruments 201a and 201b that are in the field of view of the endoscope 201d. With this, the operator can easily grasp the positional relationships between the distal ends of the surgical instruments 201a and 201b located in the field of view of the endoscope 201d based on the display of the surgical instrument position display area 324. Further, in the out-of-field portion 3242 of the surgical instrument position display area 324, the identification information of the surgical instrument 201c is displayed, to indicate the direction of the position of the surgical instrument 201c located outside the field of view of the endoscope 201d with respect to the field of view of the endoscope 201d. In the example illustrated in FIG. 5, the distal end of the surgical instrument 201a is located at the right side of the center of the field of view of the endoscope 201d, and the distal end of the surgical instrument 201b is located at the left side of the center of the field of view of the endoscope 201d. The distal end of the surgical instrument 201c is located outside of the field of view of the endoscope 201d at the lower left side therefrom. In this case, as illustrated in FIG. 7, the first identification information 324a indicating the surgical instrument 201a is displayed at the right side of the center of the in-field portion 3241 of the surgical instrument position display area 324, and the third identification information 324b indicating the surgical instrument 201b is displayed at the left side of the center of the in-field portion 3241 of the surgical instrument position display area 324. Further, the second identification information 324c indicating the surgical instrument 201c is displayed at the lower left side in the out-of-field portion 3242 of the surgical instrument position display area 324. Even in a case where the surgical instrument 201c is located farther from the field of view in the direction of the lower left side than the case illustrated in FIG. 5, the second identification information 324c indicating the surgical instrument 201c is displayed at the lower left side in the out-of-field portion 3242 of the surgical instrument position display area 324, in the same way as or similar way to the example illustrated in FIG. 5. In this case, the size of the second identification information 324c may be displayed smaller than the size of the second identification information 324c illustrated in FIG. 7, reflecting the distance from the field of view of the endoscope 201d to the surgical instrument outside of the field of view of the endoscope 201d. Note that in a case where the area of the out-of-field portion 3242 of the surgical instrument position display area 324 is larger than the example illustrated in FIG. 7, the displayed position of the second identification information 324c may be changed in response to the distance from the field of view of the endoscope 201d to the surgical instrument outside of the field of view of the endoscope 201d.

The above explanations illustrate two key aspects of the present invention in general that synergistically work together and significantly improve the control and operation of the surgical system 400. The vital operation-related information conditioned by the control apparatus 6 and displayed on the display device 3 of the system's remote control apparatus 100 are characterized by having the distinct surgical instrument position display area 324 that basically is a highly useful "map" showing the operator at once, where with respect to the field-of-view of the endoscope 201d the surgical instruments/medical equipment 201 of the patient-side apparatus 200 are. The operator is thus always fully informed of the whereabouts of the surgical instruments/ medical equipment 201, which may perhaps be in an idle position or the like. The operator can thus more efficiently control and operate the patient-side apparatus 200 of surgical system 400 via the remote control apparatus 100, since knowing well, where all instruments/equipment are placed and in what directions they have to be best moved for the next treatment step to be carried out. This more efficient control and operation in turn means any equally more efficient operation of the surgical system as a technical entity as such. The further aspect facilitating all of that is that the instrument map, i.e., the surgical instrument position display area 324, is so placed on the display (GUI) that it does not overlap or interfere with the other surgical instrument specific information already displayed there, which further contributes eventually to a higher operational efficiency and effectiveness. As it can be understood from the above explanations, that the display related components of the surgical system and the remote control apparatus being part of the surgical system according to the present invention and as detailed above and below produce a distinct technical effect in that they credibly assist the operator (surgeon) in performing a technical task, i.e., the control and operation of the surgical system, i.e., its patient-side apparatus 200 with its surgical instruments and medical equipment by means of a continued and guided human-machine interaction process that is embodied by the improved way of showing vital information on the display device 3 of the remote control apparatus 100 that is controlled via the specifically configured control apparatus 6. Said assistance to the operator in performing the control and operation of the surgical instruments is objectively, reliably and causally linked to the way the vital information is conditioned and depicted by the display-related components of the surgical system 400 and its remote control apparatus 100, respectively. The same consequently applies also to the surgical system operation method of the present invention and disclosure but not being part of the claimed invention.

Here, the control apparatus 6 obtains the position of each of the surgical instruments 201a to 201c based on the posture and the position of the corresponding manipulator 201. Further, the control apparatus 6 obtains the imaging direction of the endoscope 201d based on the posture and the position of the corresponding manipulator 201. Further, the control apparatus 6 obtains the field angle (field of view range) of the endoscope 201d based on the zoom state of the endoscope 201d. For example, the control apparatus 6 obtains the field angle (field of view range) of the endoscope 201d with reference to values of the mechanical mechanism (lens, etc.) of the endoscope 201d. Then, the control apparatus 6 obtains the coordinates of the distal end of each of the surgical instruments 201a to 201c with respect to the field of view of the endoscope 201d, based on the information on the field of view of the endoscope 201d, the posture and the position of the endoscope 201d, and the positions of the manipulators 201.

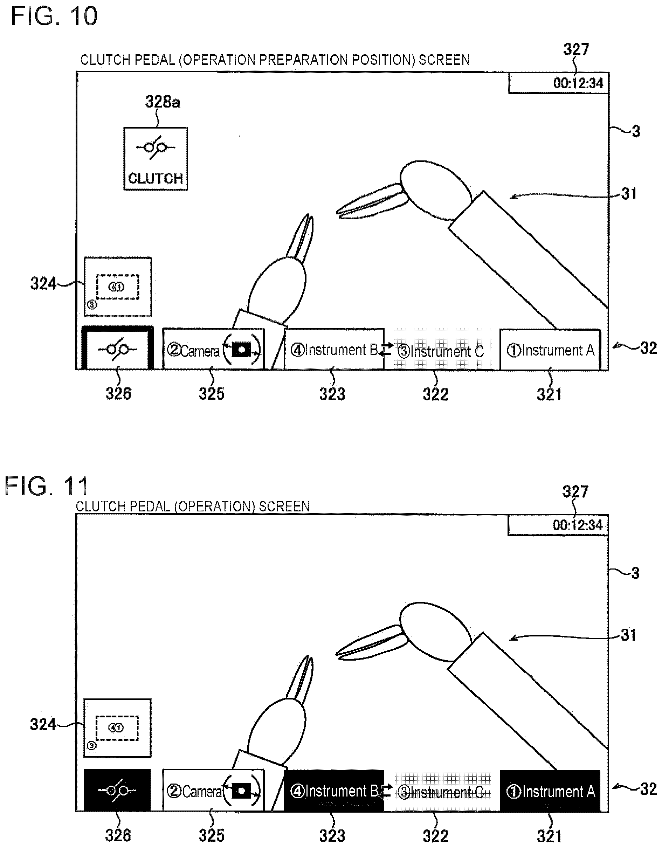

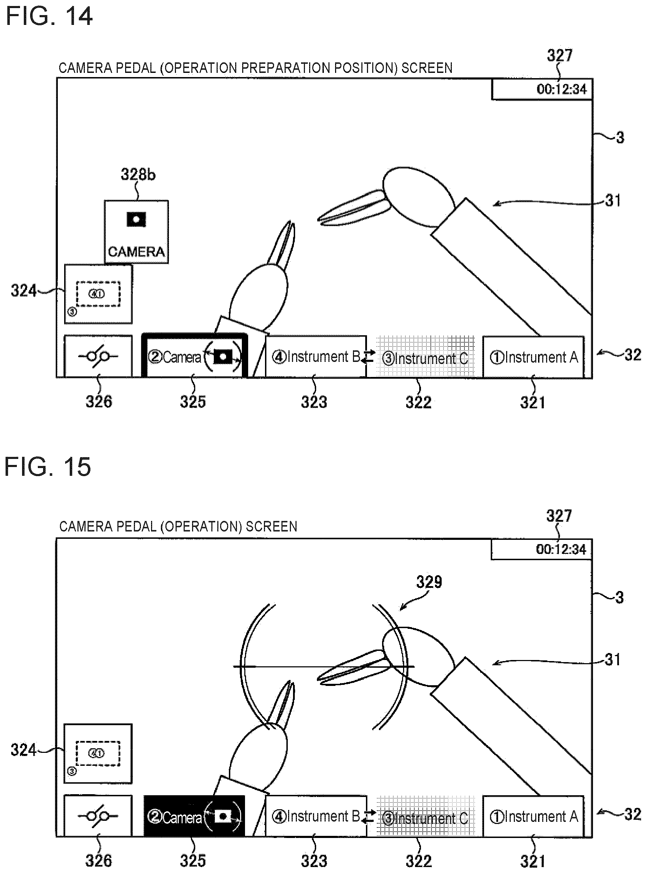

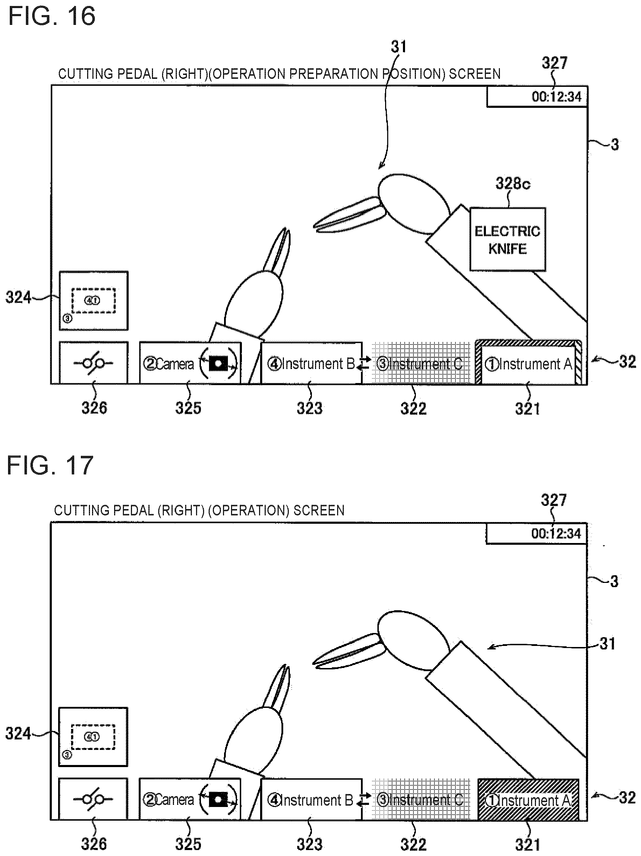

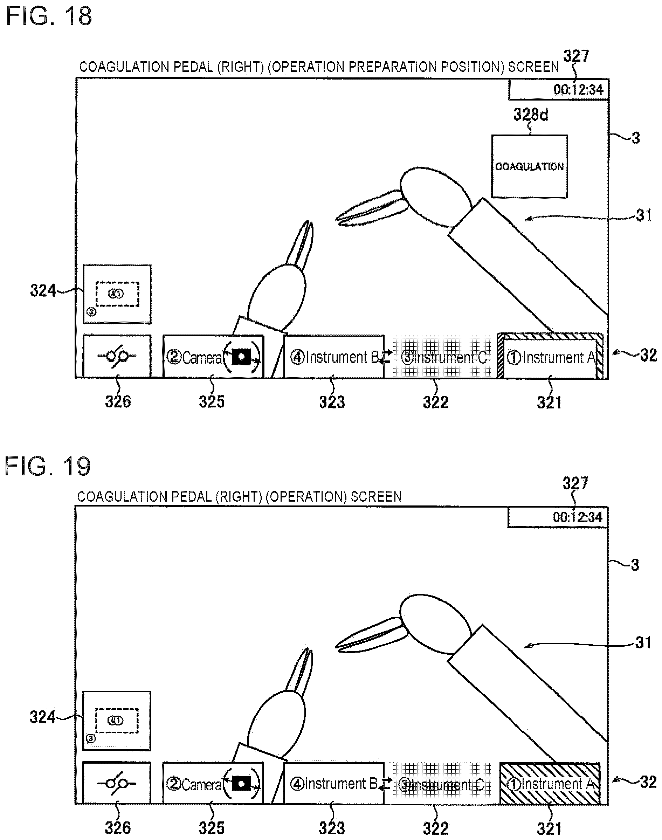

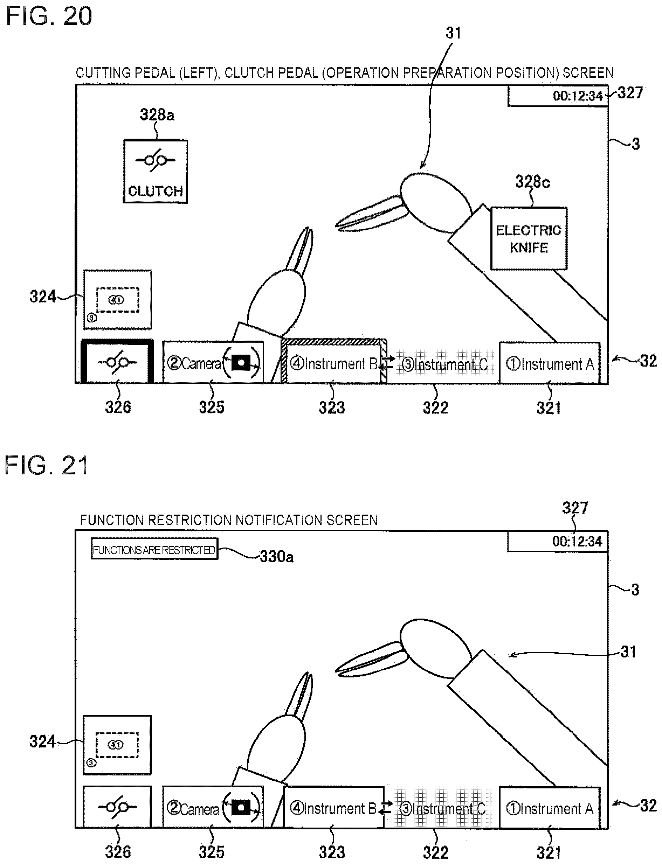

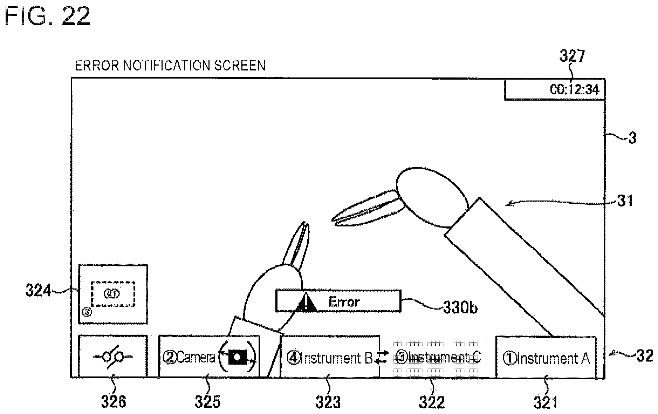

As illustrated in FIG. 6, the graphical user interface 32 displayed on the display device 3 includes the first area 321, the second area 322, the third area 323, the surgical instrument position display area 324, a fourth area 325, and a fifth area 326. Additionally, the graphical user interface 32 includes a status area 327. Moreover, the graphical user interface 32 includes a pop-up area 328a (see FIG. 10), a pop-up area 328b (see FIG. 14), a pop-up area 328c (see FIG. 16), and a pop-up area 328d (see FIG. 18). Furthermore, the graphical user interface 32 includes a central area in which a level 329 (see FIG. 15) for the endoscope 201d is displayed. The graphical user interface 32 also includes an error notification area 330a (see FIG. 21) and an error notification area 330b (see FIG. 22).

As illustrated in FIG. 6, the graphical user interface 32 includes the first, second, and third areas 321, 322, and 323 arranged in line in a lower end region of a display area (or a screen) of the display device 3, and also includes the surgical instrument position display area 324 in the vicinity of the line of the first, second, and third areas 321, 322, and 323. Note that the first, second, and third areas 321, 322, and 323 may be arranged side by side in line in an upper end region of the display area of the display device 3. This allows the display positions of the first, second, and third areas 321, 322, and 323 and the surgical instrument position display area 324 to be close to each other, so there is no need to move the line of sight significantly when checking the information displayed in the areas. Therefore, it is possible to prevent the visibility of the graphical user interface 32 from decreasing. Further, the first, second, third, fourth, and fifth areas 321, 322, 323, 325, and 326 are arranged side by side in this order from right to left in the lower end region of the display area of the display device 3. The surgical instrument position display area 324 is arranged on the upper side of the fifth area 326 in the vicinity of the left end of the display area.

The pop-up areas 328a and 328b of the graphical user interface 32 are arranged on the left side in the right-left direction and near the center in the up-down direction of the display device 3. The pop-up areas 328c and 328d of the graphical user interface 32 are arranged on the right side in the right-left direction and near the center in the up-down direction of the display device 3. The status area 327 and the error notification area 330a of the graphical user interface 32 are arranged in the upper end region of the display device 3. The error notification area 330b is arranged above the second area 322 of the graphical user interface 32.

The first area 321 displays the type of the surgical instrument 201a being operated by the right-hand operation handle 1a. The second area 322 displays the type of the surgical instrument 201c for replacement. The third area 323 displays the type of the surgical instrument 201b being operated by the left-hand operation handle 1b. Thus, the operator O can easily grasp the types of the surgical instrument 201a being operated by the operation handle 1a and surgical instrument 201b being operated by the operation handle 1b, and the type of the surgical instrument 201c for changing the type of the operation.

The second area 322 is arranged between the first area 321 and the third area 323. The information in the second area 322 is displayed with paler color than the information in the first area 321 and third area 323. In other words, the information in the second area 322 is displayed to be more inconspicuous than the information in the first area 321 and third area 323. Note that as long as the first area 321 is arranged on the right side of the third area 323, the second area 322 may be arranged on the right side of the first area 321 or may be arranged on the left side of the second area 322.

As illustrated in FIG. 6, in the first area 321, the first identification information 321a indicating the surgical instrument 201a is displayed. In the second area 322, the second identification information 322a indicating the surgical instrument 201c is displayed. In the third area 323, the third identification information 323a indicating the surgical instrument 201b is displayed. Further, to indicates the positions of the surgical instruments 201a, 201b, and 201c with respect to the field of view of the endoscope 201d, first identification information 324a, second identification information 324c, and third identification information 324b are displayed in the surgical instrument position display area 324 as illustrated in FIG. 7. With this configuration, the first identification information 324a, the third identification information 324b, and the second identification information 324c, which are simple displays indicating the surgical instruments 201a, 201b, and 201c, respectively, can be displayed in the surgical instrument position display area 324. As a result, the first, second, and third identification information 324a, 324c, and 324b are simply displayed with being distinguishable from each other in the surgical instrument position display area 324. Thus, the visibility of the surgical instrument position display area 324 can be improved.

The first identification information 321a (324a), the second identification information 322a (324c), the third identification information 323a (324b) may include numbers, letters, or symbols which are different from each other. With this, the first identification information 321a (324a), the second identification information 322a (324c), and the third identification information 323a (324b) corresponding to the surgical instruments 201a, 201c, and 201b, respectively are distinguishable from each other in the simplified display forms. Specifically, in an example, the first identification information 321a in the first area 321 and the first identification information 324a in the surgical instrument position display area 324 are displayed with the same number (1). Also the second identification information 322a in the second area 322 and the second identification information 324c in the surgical instrument position display area 324 are displayed with the same number (3). Further, the third identification information 323a in the third area 323 and the third identification information 324b in the surgical instrument position display area 324 are displayed with the same number (4). Note that the first identification information 321a (324a), the second identification information 322a (324c), and the third identification information 323a (324b) may be displayed in different colors, respectively. Also the first identification information 321a (324a), the second identification information 322a (324c), and the third identification information 323a (324b) may be displayed with numbers, letters, or symbols corresponding to the manipulators 201, which supports the surgical instruments 201a, 201c, and 201b, respectively.

As illustrated in FIG. 8, it is also possible to hide the surgical instrument position display area 324. Specifically, by operating a display switch button 51a in the touch panel 51, it is possible to show or hide the surgical instrument position display area 324. With this, the surgical instrument position display area 324 can be hide if necessary, so as to enlarge an area of display of the image captured by the endoscope 201d. Note that the display switch button 51a may be an example of a "display switch part". The display switch part to show or hide the surgical instrument position display area 324 may be provided out of the touch panel 51.

In the fourth area 325, information indicating the endoscope 201d is displayed. The fourth area 325 is provided on the left side of the third area 323.

In the fifth area 326, information relating to the clutch function. The fifth area 326 is provided on the left side of the fourth area 325. In the fifth area 326, an operation state of the clutch pedal 23 is displayed.

In the status area 327, the status of the surgical system 400 is displayed. For example, the status area 327 displays duration of surgery procedure. The status area 327 also displays brightness adjustment information and contrast adjustment information of the display device 3.

The pop-up areas 328a to 328d are arranged in positions different from those of the first, second, and third areas 321, 322, and 323. The pop-up areas 328a to 328d display information, based on a fact that at least one of the sensors 26 detects setting of the foot of the operator O at the operation preparation position of the corresponding foot pedal 20.

As illustrated in FIG. 10, the pop-up area 328a displays the information on the clutch pedal 23 when the foot of the operator O is set at the operation preparation position of the clutch pedal 23. The pop-up area 328a displays the information for a predetermined time (e.g., three seconds). That is, the display of the information in the pop-up area 328a ends when the foot of the operator O is moved from the operation preparation position of the clutch pedal 23, when the clutch pedal 23 is operated, or after the predetermined time has been elapsed.

As illustrated in FIG. 14, the pop-up area 328b displays information on the camera pedal 24 when the foot of the operator O is set at the operation preparation position of the camera pedal 24. The pop-up area 328b display the information for a predetermined time (e.g., three seconds). That is, the display of the information in the pop-up area 328b ends when the foot of the operator O is moved from the operation preparation position of the camera pedal 24, when the camera pedal 24 is operated, or after the predetermined time has been elapsed.

As illustrated in FIG. 16, the pop-up area 328c displays information on the cutting pedals 22a or 22b when the foot of the operator O is set at the operation preparation position of the cutting pedal 22a or 22b. The pop-up area 328c displays the information for a predetermined time (e.g., three seconds). That is, the display of the information in the pop-up area 328c ends when the foot of the operator O is moved from the operation preparation position of the cutting pedal 22a or 22b, when the cutting pedal 22a or 22b is operated, or after the predetermined time has been elapsed.

As illustrated in FIG. 18, the pop-up area 328d displays information on the coagulation pedals 21a or 21b when the foot of the operator O is set at the operation preparation position of the coagulation pedal 21a or 21b. The pop-up area 328d displays the information for a predetermined time (e.g., three seconds). That is, the display of the information in the pop-up area 328d ends when the foot of the operator O is moved from the operation preparation position of the coagulation pedal 21a or 21b, when the coagulation pedal 21a or 21b is operated, or after the predetermined time has been elapsed.

As illustrated in FIG. 15, the level 329 for the endoscope 201d is displayed on the display device 3 when the camera pedal 24 is operated. Accordingly, it is possible to easily change the orientation of the endoscope 201d through operation referring to the level 329.

The control apparatus 6 is configured to change the display form of a corresponding area out of the first area 321, the third area 323, and the fourth area 325 based on a fact that at least one of the sensors 26 detects setting of the foot of the operator O at the operation preparation position of at least one foot pedal 20 out of the right-side foot pedals (the coagulation pedal 21b and the cutting pedal 22b), the left-side foot pedals (the coagulation pedal 21a and the cutting pedal 22a), and the camera pedal 24.

The changing of the display form of the area includes at least one of display color change, inverted display, display change of the area frame line, and zoom/zoom-out of the area.

The first area 321, third area 323, surgical instrument position display area 324, fourth area 325, and fifth area 326 of the graphical user interface 32 are configured to display the operation states of the right-side foot pedals (the coagulation pedal 21b and the cutting pedal 22b), the left-side foot pedals (the coagulation pedal 21a and the cutting pedal 22a), the switch pedal 25, the camera pedal 24, and the clutch pedal 23, respectively. Thus, the operator O can easily grasp the operation states of the foot pedals 20.

Specifically, the first area 321 displays the information on the right-side foot pedals (the coagulation pedal 21b and the cutting pedal 22b) in addition to the information on the surgical instrument 201a. For example, when the foot of the operator O is set at the operation preparation position of the coagulation pedal 21b or the cutting pedal 22b, the frame line of the first area 321 is displayed with the color of the corresponding foot pedal 20 (the coagulation pedal 21b or the cutting pedal 22b). When the coagulation pedal 21b or the cutting pedal 22b is operated, the first area 321 displays the information on the coagulation pedal 21b or the cutting pedal 22b such that the display is distinguished from that in the case where the foot of the operator O is set at the operation preparation position. For example, when the coagulation pedal 21b or the cutting pedal 22b is operated, the entirety of the first area 321 is displayed with the color of the corresponding foot pedal 20 (the coagulation pedal 21b or the cutting pedal 22b).

In other words, when the coagulation pedal 21b is operated, the first area 321 displays the information on the coagulation pedal 21b. When the cutting pedal 22b is operated, the first area 321 displays the information on the cutting pedal 22b.

The third area 323 displays the information on the left-side foot pedals (the coagulation pedal 21a and the cutting pedal 22a) in addition to the information on the surgical instrument 201b. For example, when the foot of the operator O is set at the operation preparation position of the coagulation pedal 21a or the cutting pedal 22a, the frame line of the third area 323 is displayed with the color of the corresponding foot pedal 20 (the coagulation pedal 21a or the cutting pedal 22a). When the coagulation pedal 21a or the cutting pedal 22a is operated, the third area 323 displays the information on the coagulation pedal 21a or the cutting pedal 22a such that the display is distinguished from that in the case where the foot of the operator O is set at the operation preparation position. For example, when the coagulation pedal 21a or the cutting pedal 22a is operated, the entirety of the third area 323 is displayed with the color of the corresponding foot pedal 20 (the coagulation pedal 21a or the cutting pedal 22a).

Further, the surgical instrument position display area 324 displays the information on the switch pedal 25 in addition to the information relating to the positional relationships of the surgical instruments 201a to 201c. For example, when the switch pedal 25 is operated, the background color of and the text color in the surgical instrument position display area 324 are inverted.

The fourth area 325 displays the information on the camera pedal 24 in addition to the information on the endoscope 201d. For example, when the foot of the operator O is set at the operation preparation position of the camera pedal 24, the frame line of the fourth area 325 is emphasized. Further, when the camera pedal 24 is operated, the fourth area 325 displays the information on the camera pedal 24 such that the display is distinguished from that in the case where the foot of the operator O is set at the operation preparation position. For example, when the camera pedal 24 is operated, the background color and the text color of the fourth area 325 are inverted.

The fifth area 326 displays the information on the clutch pedal 23. For example, when the foot of the operator is set at the operation preparation position of the clutch pedal 23, the frame line of the fifth area 326 is emphasized. Further, when the clutch pedal 23 is operated, the fifth area 326 displays the information on the clutch pedal 23 such that the display is distinguished from that in the case where the foot of the operator O is set at the operation preparation position. For example, when the clutch pedal 23 is operated, the background color of and the text color in the fifth area 326 are inverted.

Next, with reference to FIGS. 9 to 22, an example of the graphical user interface 32 displayed based on the operation of the foot pedals 20 by the operator O.

As illustrated in FIG. 9, when the switch pedal 25 is operated, the surgical instrument operated by the left hand-operation handle 1b is switched from the surgical instrument 201b to the surgical instrument 201c. In this case, the background colors and the text colors of the second area 322 and the third area 323 are inverted. Thus, the operator O can easily recognize the switching of the surgical instruments.

As illustrated in FIG. 10, when the foot of the operator O is set at the operation preparation position of the clutch pedal 23, the frame line of the fifth area 326 is emphasized. The information on the clutch pedal 23 is popped up and displayed in the pop-up area 328a. Even when the foot of the operator O continues to be set at the operation preparation position of the clutch pedal 23, the display of the pop-up area 328a ends after the predetermined time has been elapsed. In this case, the emphasized display of the frame line of the fifth area 326 continues.

As illustrated in FIG. 11, when the clutch pedal 23 is operated, the control-related connections between the operation handles 1a and 1b and the manipulators 201 are temporarily disconnected. In this case, the background color and the text color of the fifth area 326 displaying the information on the clutch pedal 23 are inverted. Additionally, the background color and the text color of the first area 321 displaying the information on the surgical instrument 201a in which the control-related connection is temporarily disconnected are inverted. Moreover, the background color and the text color of the third area 323 displaying the information on the surgical instrument 201b in which the control-related connection is temporarily disconnected are inverted. When the foot of the operator O goes back to the operation preparation position after the operation, the frame line of the fifth area 326 is emphasized as illustrated in FIG. 10.

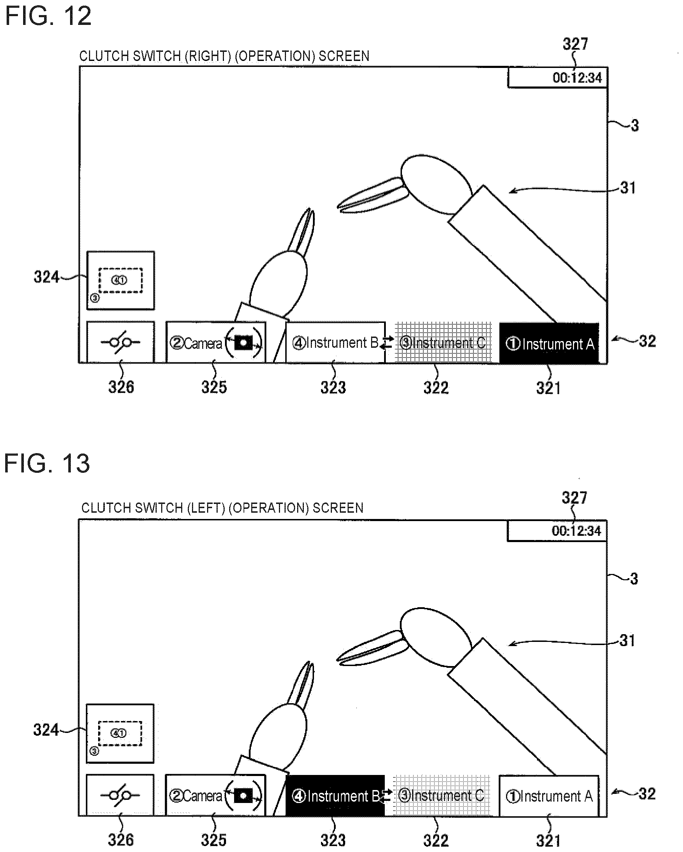

As illustrated in FIG. 12, when the clutch switch 11 of the right hand-operation handle 1a is operated, the control-related connection between the operation handle 1a and the manipulator 201 provided with the surgical instrument 201a is temporarily disconnected. In this case, the background color and text color of the first area 321 displaying the information on the surgical instrument 201a in which the control-related connection is temporarily disconnected are inverted.

As illustrated in FIG. 13, when the clutch switch 11 of the left hand-operation handle 1b is operated, the control-related connection between the operation handle 1b and the manipulator 201 provided with the surgical instrument 201b is temporarily disconnected. In this case, the background color and the text color of the third area 323 displaying the information on the surgical instrument 201b in which the control-related connection is temporarily disconnected are inverted.

As illustrated in FIG. 14, when the foot of the operator O is set at the operation preparation position of the camera pedal 24, the frame line of the fourth area 325 is emphasized. Additionally, the information on the camera pedal 24 is popped up and displayed in the pop-up area 328b. Even when the foot of the operator O continues to be set at the operation preparation position of the camera pedal 24, the display of the pop-up area 328b ends after the predetermined time has been elapsed. In this case, the emphasized display of the frame line of the fourth area 325 continues.

As illustrated in FIG. 15, when the camera pedal 24 is operated, the position and orientation of the endoscope 201d can be controlled by the operation handles 1a and 1b. In this process, the background color and the text color of the fourth area 325 displaying the information on the camera pedal 24 are inverted. Additionally, the level 329 for the endoscope 201d is displayed in the central area.

As illustrated in FIG. 16, when the foot of the operator O is set at the operation preparation position of the cutting pedal 22b corresponding to the surgical instrument 201a being operated by the right hand, the frame line of the first area 321 is emphasized. Specifically, more than left half of the frame line is emphasized by the second color of the cutting pedal 22b. The rest of the frame line is emphasized by the first color of the coagulation pedal 21b. Additionally, the information on the cutting pedal 22b is popped up and displayed in the pop-up area 328c. Even when the foot of the operator O continues to be set at the operation preparation position of the cutting pedal 22b, the display of the pop-up area 328c ends after the predetermined time has been elapsed. In this case, the emphasized display of the frame line of the first area 321 continues.

Likewise, the frame line of the third area 323 is emphasized also when the foot of the operator O is set at the operation preparation position of the cutting pedal 22a corresponding to the surgical instrument 201b being operated by the left hand.

As illustrated in FIG. 17, when the cutting pedal 22b is operated, predetermined voltage is applied to the surgical instrument 201a to enable cutting of the surgery site. In this process, the background of the first area 321 displaying the information on the cutting pedal 22b is displayed with the second color of the cutting pedal 22b.

Likewise, also when the cutting pedal 22a is operated, the background of the third area 323 displaying the information on the cutting pedal 22a is displayed with the second color of the cutting pedal 22a.

As illustrated in FIG. 18, when the foot of the operator O is set at the operation preparation position of the coagulation pedal 21b corresponding to the surgical instrument 201a being operated by the right hand, the frame line of the first area 321 is emphasized. Specifically, more than right half of the frame line is emphasized by the first color of the coagulation pedal 21b. The rest of the frame line is emphasized by the second color of the cutting pedal 22b. Additionally, the information on the coagulation pedal 21b is popped up and displayed in the pop-up area 328d. Even when the foot of the operator O continues to be set at the operation preparation position of the coagulation pedal 21b, the display of the pop-up area 328d ends after the predetermined time has been elapsed. In this case, the emphasized display of the frame line of the first area 321 continues.

Likewise, the frame line of the third area 323 is emphasized also when the foot of the operator O is set at the operation preparation position of the coagulation pedal 21a corresponding to the surgical instrument 201b being operated by the left hand.

As illustrated in FIG. 19, when the coagulation pedal 21b is operated, predetermined voltage is applied to the surgical instrument 201a to enable cutting of the surgery site. In this process, the background of the first area 321 displaying the information on the coagulation pedal 21b is displayed with the first color of the coagulation pedal 21b.

Likewise, also when the coagulation pedal 21a is operated, the background of the third area 323 displaying the information on the coagulation pedal 21a is displayed with the first color of the coagulation pedal 21a.

As illustrated in FIG. 20, when the foot of the operator O is set at the operation preparation position of the cutting pedal 22a corresponding to the surgical instrument 201b being operated by the left hand and the other foot of the operator O is set at the operation preparation position of the clutch pedal 23, the frame line of the third area 323 is emphasized. Specifically, more than left half of the frame line is emphasized by the second color of the cutting pedal 22a. The rest of the frame line is emphasized by the first color of the coagulation pedal 21a. Additionally, the information on the cutting pedal 22a is popped up and displayed in the pop-up area 328c. Moreover, the frame line of the fifth area 326 is emphasized. The information on the clutch pedal 23 is popped up and displayed in the pop-up area 328a.

As illustrated in FIG. 21, when the functions are restricted, a notification to indicate that the functions are restricted is displayed in the error notification area 330a. When the cutting pedal 22a (22b) or the coagulation pedals 21a (21b) is operated while a surgical instrument in which the functions of cutting and coagulating are not set is used, a notification to indicate that unfunctional operation is tried to be executed is popped up and displayed on the upper side of the area corresponding to the surgical instrument. In this case, no voltage is applied to the surgical instrument even if the cutting pedal 22a (22b) or the coagulation pedals 21a (21b) is operated.

As illustrated in FIG. 22, when an error is notified, the error notification is displayed in the error notification area 330b.

## Claims

1. A surgical system (400) comprising:
a patient-side apparatus (200) including:
a first manipulator (201) that supports a first surgical instrument (201a);
a second manipulator (201) that supports a second surgical instrument (201b);
a third manipulator (201) that supports a third surgical instrument (201c); and
a fourth manipulator (201) that supports an endoscope (201d);
a remote-control apparatus (100) for operation of the first to fourth manipulators, and including a display device (3) configured to display an image (31) captured by the endoscope; and
a control apparatus (6) configured and arranged to display, on the display device (3), a graphical user interface (32) overlapped with the image (31) captured by the endoscope (201d),
**characterized in that** the graphical user interface including a first area (321) that indicates information on the first surgical instrument (201a), a second area (322) that indicates information on the third surgical instrument (201c), a third area (323) that indicates information on the second surgical instrument (201b), which are arranged in one of an upper end portion and a lower end portion in a display area of the display device (3) and further including, at a position different from that of the one of the upper end portion and the lower end portion in the display area, a surgical instrument position display area (324) that indicates positional relationships of the first, second, third surgical instruments (201a, 201b, 201c) with respect to a field of view of the endoscope (201d).

2. The surgical system according to claim 1, wherein
the lower end portion is an area including a lower end of the display area of the display device,
the upper end portion is an area including an upper end of the display area of the display device,
the first, second, and third areas are arranged in line in the one of the upper end portion and the lower end portion, and
the surgical instrument position display area is provided on an inner side of the display area from the one of the upper end portion and the lower end portion.

3. The surgical system according to claim 1 or 2, wherein
the surgical instrument position display area is provided in an area other than a right end portion or a left end portion of the display device.

4. The surgical system according to claim 1, 2 or 3, wherein
the surgical instrument position display area includes an in-field portion (3241) corresponding to the field of view of the endoscope and an out-of-field portion (3242) corresponding to an outside of the field of view of the endoscope around the field of view, and
the out-of-field portion displays, when one or more of the first, second, third surgical instruments are located outside the field of view of the endoscope, information of each of the one or more of the first, second, third surgical instruments that are located outside the field of view of the endoscope.

5. The surgical system according to claim 4, wherein
the information of each of the one or more of the first, second, third surgical instruments that are located outside the field of view of the endoscope is displayed at a position in the out-of-field portion that corresponds to a direction from the field of view of the endoscope to the surgical instrument, to indicate the position of the surgical instrument with respect to the field of view of the endoscope.

6. The surgical system according to claim 4, wherein
the information of each of one or more of the first, second, third surgical instruments that are located in the field of view of the endoscope is displayed at a position in the in-field portion of the surgical instrument position display area corresponding to a position of a distal end of the surgical instrument in the field of view of the endoscope.

7. The surgical system according to any one of claims 1 to 6, wherein
the graphical user interface includes the surgical instrument position display area in the vicinity of a line of the first, second, and third areas which are arranged in line.

8. The surgical system according to any one of claims 1 to 7, wherein
the first area displays a first identification information indicating the first surgical instrument,
the second area displays a second identification information indicating the third surgical instrument,
the third area displays a third identification information indicating the second surgical instrument, and
the surgical instrument position display area displays the first, second, and third identification information so as to indicate a position of the first, second, and third surgical instruments with respect to the field of view of the endoscope.

9. The surgical system according to claim 8, wherein
the first, second, and third identification information includes numbers, letters, or symbols which are different from each other.

10. The surgical system according to any one of claims 1 to 9, wherein
in the graphical user interface, the first, second, and third areas are arranged in line from right to left, and
the graphical user interface further includes a fourth area arranged on a left side of the third area, wherein the fourth area displays information on the endoscope.

11. The surgical system according to any one of claims 1 to 10, further comprising
an operation table (300) for a patient placed in operational range of the patient-side apparatus.

12. The surgical system (400)according to any one of claims 1 to 11, wherein
the remote control apparatus (100) includes:
a first operation handle (1a) for right hand to operate the first surgical instrument; and
a second operation handle (1b) for left hand to operate the second surgical instrument.

13. The surgical system according to claim 10, wherein the remote control apparatus (100) further comprises:
an operation pedal section (2) including: a first foot pedal (21b, 22b) configured to execute a function relating to the first surgical instrument; a second foot pedal (21a, 22a) configured to execute a function relating to the second surgical instrument; a third foot pedal (24) configured to execute a function relating to the endoscope; and a fourth foot pedal (23) configured to execute a clutch function, wherein
the graphical user interface further includes a fifth area arranged on a left side of the fourth area, wherein the fifth area displays information on the clutch function, preferably wherein
the first, third, fourth, and fifth areas in the graphical user interface are configured to display operation states of the first, second, third, fourth foot pedals, respectively.

14. The surgical system according to any one of claims 1 to 12, wherein the remote control apparatus (100) further comprises:
an operation pedal section including: a first foot pedal (21b, 22b) configured to execute a function relating to the first surgical instrument; a second foot pedal (21a, 22a) configured to execute a function relating to the second surgical instrument; a third foot pedal (24) configured to execute a function relating to the endoscope; and a fourth foot pedal (25) configured to change one of the first surgical instrument to be operated by the first operation handle and the second surgical instrument to be operated by the second operation handle to the third surgical instrument.

15. The surgical system according to any one of claims 1 to 14, wherein the remote control apparatus further comprises:
a display switch part (51a) configured to switch between showing and hiding the surgical instrument position display area.

## Patentansprüche

1. Chirurgisches System (400), umfassend:
eine patientenseitige Einrichtung (200), aufweisend:
einen ersten Manipulator (201), der ein erstes chirurgisches Instrument (201a) trägt;
einen zweiten Manipulator (201), der ein zweites chirurgisches Instrument (201b) trägt;
einen dritten Manipulator (201), der ein drittes chirurgisches Instrument (201c) trägt; und
einen vierten Manipulator (201), der ein Endoskop (201d) trägt;
eine Fernsteuerungseinrichtung (100) für die Bedienung des ersten bis vierten Manipulators und, die eine Anzeigevorrichtung (3) einschließt, die so konfiguriert ist, dass sie ein von dem Endoskop aufgenommenes Bild (31) anzeigt; und
eine Steuereinrichtung (6), die so konfiguriert und angeordnet ist, dass sie auf der Anzeigevorrichtung (3) eine grafische Benutzeroberfläche (32) anzeigt, die mit dem von dem Endoskop (201d) aufgenommenen Bild (31) überlappt,
**dadurch gekennzeichnet, dass** die grafische Benutzeroberfläche einen ersten Bereich (321), der Informationen über das erste chirurgische Instrument (201a) angibt, einen zweiten Bereich (322), der Informationen über das dritte chirurgische Instrument (201c) angibt, und einen dritten Bereich (323), der Informationen über das zweite chirurgische Instrument (201b) angibt, einschließt, die in einem von einem oberen Endabschnitt und einem unteren Endabschnitt in einem Anzeigebereich der Anzeigevorrichtung (3) angeordnet sind, und weiter an einer Position, die sich von derjenigen des oberen Endabschnitts oder des unteren Endabschnitts in dem Anzeigebereich unterscheidet, einen Anzeigebereich (324) für die Position des chirurgischen Instruments einschließt, der die Positionsbeziehungen des ersten, zweiten und dritten chirurgischen Instruments (201a, 201b, 201c) in Bezug auf ein Sichtfeld des Endoskops (201d) angibt.

2. Chirurgisches System nach Anspruch 1, wobei
der untere Endabschnitt ein Bereich ist, der ein unteres Ende des Anzeigebereichs der Anzeigevorrichtung einschließt,
der obere Endabschnitt ein Bereich ist, der ein oberes Ende des Anzeigebereichs der Anzeigevorrichtung einschließt,
der erste, der zweite und der dritte Bereich in einem von dem oberen Endabschnitt und dem unteren Endabschnitt in einer Linie angeordnet sind, und
der Anzeigebereich für die Position des chirurgischen Instruments an einer Innenseite des Anzeigebereichs von dem einen von dem oberen Endabschnitt und dem unteren Endabschnitt vorgesehen ist.

3. Chirurgisches System nach Anspruch 1 oder 2, wobei
der Anzeigebereich für die Position des chirurgischen Instruments in einem anderen Bereich als einem rechten Endabschnitt oder einem linken Endabschnitt der Anzeigevorrichtung vorgesehen ist.

4. Chirurgisches System nach Anspruch 1, 2 oder 3, wobei
der Anzeigebereich für die Position des chirurgischen Instruments einen Abschnitt (3241) innerhalb des Feldes, der dem Sichtfeld des Endoskops entspricht, und einen Abschnitt (3242) außerhalb des Feldes einschließt, der einer Außenseite des Sichtfeldes des Endoskops um das Sichtfeld herum entspricht, und
der Abschnitt außerhalb des Feldes, wenn eines oder mehrere des ersten, zweiten, dritten chirurgischen Instruments sich außerhalb des Sichtfeldes des Endoskops befinden, Informationen über jedes des einen oder der mehreren des ersten, zweiten, dritten chirurgischen Instruments anzeigt, die sich außerhalb des Sichtfeldes des Endoskops befinden.

5. Chirurgisches System nach Anspruch 4, wobei
die Informationen über jedes des einen oder der mehreren des ersten, zweiten, dritten chirurgischen Instruments, die sich außerhalb des Sichtfeldes des Endoskops befinden, an einer Position in dem Abschnitt außerhalb des Feldes angezeigt werden, die einer Richtung von dem Sichtfeld des Endoskops zu dem chirurgischen Instrument entspricht, um die Position des chirurgischen Instruments in Bezug auf das Sichtfeld des Endoskops anzugeben.

6. Chirurgisches System nach Anspruch 4, wobei
die Informationen über jedes von einem oder mehreren des ersten, zweiten, dritten chirurgischen Instruments die sich in dem Sichtfeld des Endoskops befinden, an einer Position in dem Abschnitt innerhalb des Feldes des Anzeigebereichs für die Position des chirurgischen Instruments angezeigt werden, die einer Position eines distalen Endes des chirurgischen Instruments in dem Sichtfeld des Endoskops entspricht.

7. Chirurgisches System nach einem der Ansprüche 1 bis 6, wobei
die graphische Benutzeroberfläche den Anzeigebereich für die Position des chirurgischen Instruments in der Nähe einer Linie des ersten, des zweiten und des dritten Bereichs, die in einer Linie angeordnet sind, einschließt.

8. Chirurgisches System nach einem der Ansprüche 1 bis 7, wobei
der erste Bereich eine erste Identifikationsinformation anzeigt, die das erste chirurgische Instrument angibt,
der zweite Bereich eine zweite Identifikationsinformation anzeigt, die das dritte chirurgische Instrument angibt,
der dritte Bereich eine dritte Identifikationsinformation anzeigt, die das zweite chirurgische Instrument angibt, und
der Anzeigebereich für die Position des chirurgischen Instruments die erste, zweite und dritte Identifikationsinformation anzeigt, um damit eine Position des ersten, zweiten und dritten chirurgischen Instruments in Bezug auf das Sichtfeld des Endoskops anzugeben.

9. Chirurgisches System nach Anspruch 8, wobei
die erste, zweite und dritte Identifikationsinformation Zahlen, Buchstaben oder Symbole einschließen, die voneinander verschieden sind.

10. Chirurgisches System nach einem der Ansprüche 1 bis 9, wobei
in der grafischen Benutzeroberfläche der erste, zweite und dritte Bereich in einer Linie von rechts nach links angeordnet sind, und
die grafische Benutzeroberfläche weiter einen vierten Bereich einschließt, der auf einer linken Seite des dritten Bereichs angeordnet ist, wobei der vierte Bereich Informationen über das Endoskop anzeigt.

11. Chirurgisches System nach einem der Ansprüche 1 bis 10, weiter umfassend
einen Operationstisch (300) für einen Patienten, der im Wirkbereich der patientenseitigen Einrichtung platziert ist.

12. Chirurgisches System (400) nach einem der Ansprüche 1 bis 11, wobei
die Fernsteuerungseinrichtung (100) einschließt:
einen ersten Bediengriff (1a) für die rechte Hand zur Bedienung des ersten chirurgischen Instruments; und
einen zweiten Bediengriff (1b) für die linke Hand zur Bedienung des zweiten chirurgischen Instruments.

13. Chirurgisches System nach Anspruch 10, wobei die Fernsteuerungseinrichtung (100) weiter umfasst:
einen Bedienpedalabschnitt (2), der einschließt: ein erstes Fußpedal (21b, 22b), das so konfiguriert ist, dass es eine Funktion bezüglich des ersten chirurgischen Instruments ausführt; ein zweites Fußpedal (21a, 22a), das so konfiguriert ist, dass es eine Funktion bezüglich des zweiten chirurgischen Instruments ausführt; ein drittes Fußpedal (24), das so konfiguriert ist, dass es eine Funktion bezüglich des Endoskops ausführt; und ein viertes Fußpedal (23), das so konfiguriert ist, dass es eine Kupplungsfunktion ausführt, wobei
die grafische Benutzeroberfläche weiter einen fünften Bereich einschließt, der an einer linken Seite des vierten Bereichs angeordnet ist, wobei der fünfte Bereich Informationen über die Kupplungsfunktion anzeigt, vorzugsweise wobei
der erste, dritte, vierte und fünfte Bereich in der grafischen Benutzeroberfläche so konfiguriert sind, dass sie Betriebszustände des ersten, zweiten, dritten beziehungsweise vierten Fußpedals anzeigen.

14. Chirurgisches System nach einem der Ansprüche 1 bis 12, wobei die Fernsteuerungseinrichtung (100) weiter umfasst:
einen Bedienpedalabschnitt, der einschließt: ein erstes Fußpedal (21b, 22b), das so konfiguriert ist, dass es eine Funktion bezüglich des ersten chirurgischen Instruments ausführt; ein zweites Fußpedal (21a, 22a), das so konfiguriert ist, dass es eine Funktion bezüglich des zweiten chirurgischen Instruments ausführt; ein drittes Fußpedal (24), das so konfiguriert ist, dass es eine Funktion bezüglich des Endoskops ausführt; und ein viertes Fußpedal (25), das so konfiguriert ist, dass es das erste chirurgische Instrument, das durch den ersten Bediengriff zu bedienen ist, oder das zweite chirurgische Instrument, das durch den zweiten Bediengriff zu bedienen ist, in das dritte chirurgische Instrument wechselt.

15. Chirurgisches System nach einem der Ansprüche 1 bis 14, wobei die Fernsteuerungseinrichtung weiter umfasst:
einen Anzeigeschaltteil (51a), der so konfiguriert ist, dass er zwischen dem Anzeigen und dem Verbergen des Anzeigebereichs für die Position des chirurgischen Instruments umschaltet.

## Revendications

1. Système chirurgical (400) comprenant :
un appareil (200) côté patient comportant :
un premier manipulateur (201) qui supporte un premier instrument chirurgical (201a) ;
un deuxième manipulateur (201) qui supporte un deuxième instrument chirurgical (201b) ;
un troisième manipulateur (201) qui supporte un troisième instrument chirurgical (201c) ; et
un quatrième manipulateur (201) qui supporte un endoscope (201d) ;
un appareil (100) de commande à distance pour l'actionnement des premier à quatrième manipulateurs, et comportant un dispositif d'affichage (3) configuré pour afficher une image (31) capturée par l'endoscope ; et
un appareil de commande (6) configuré et agencé pour afficher, sur le dispositif d'affichage (3), une interface utilisateur graphique (32) chevauchée par l'image (31) capturée par l'endoscope (201d),
**caractérisé en ce que** l'interface utilisateur graphique comporte une première zone (321) qui indique des informations concernant le premier instrument chirurgical (201a), une deuxième zone (322) qui indique des informations concernant le troisième instrument chirurgical (201c), une troisième zone (323) qui indique des informations concernant le deuxième instrument chirurgical (201b), qui sont agencées dans une parmi une partie d'extrémité supérieure et une partie d'extrémité inférieure dans une zone d'affichage du dispositif d'affichage (3) et comportant en outre, à une position différente de celle de ladite une parmi la partie d'extrémité supérieure et la partie d'extrémité inférieure dans la zone d'affichage, une zone (324) d'affichage de position d'instrument chirurgical qui indique des relations positionnelles des premier, deuxième, troisième instruments chirurgicaux (201a, 201b, 201c) par rapport à un champ de vision de l'endoscope (201d).

2. Système chirurgical selon la revendication 1, dans lequel
la partie d'extrémité inférieure est une zone incluant une extrémité inférieure de la zone d'affichage du dispositif d'affichage,
la partie d'extrémité supérieure est une zone incluant une extrémité supérieure de la zone d'affichage du dispositif d'affichage,
les première, deuxième et troisième zones sont agencées en ligne dans ladite une parmi la partie d'extrémité supérieure et la partie d'extrémité inférieure, et
la zone d'affichage de position d'instrument chirurgical est située sur un côté intérieur de la zone d'affichage par rapport à ladite une parmi la partie d'extrémité supérieure et la partie d'extrémité inférieure.

3. Système chirurgical selon la revendication 1 ou la revendication 2, dans lequel
la zone d'affichage de position d'instrument chirurgical est située dans une zone autre qu'une partie d'extrémité droite ou qu'une partie d'extrémité gauche du dispositif d'affichage.

4. Système chirurgical selon la revendication 1, 2 ou 3, dans lequel
la zone d'affichage de position d'instrument chirurgical comporte une partie dans-le-champ (3241) correspondant au champ de vision de l'endoscope et une partie hors-champ (3242) correspondant à un extérieur du champ de vision de l'endoscope autour du champ de vision, et
la partie hors-champ affiche, lorsqu'un ou plusieurs des premier, deuxième, troisième instruments chirurgicaux se situent à l'extérieur du champ de vision de l'endoscope, des informations de chacun desdits un ou plusieurs des premier, deuxième, troisième instruments chirurgicaux qui se situent à l'extérieur du champ de vision de l'endoscope.

5. Système chirurgical selon la revendication 4, dans lequel
les informations de chacun desdits un ou plusieurs parmi les premier, deuxième, troisième instruments chirurgicaux qui se situent à l'extérieur du champ de vision de l'endoscope sont affichées en une position située dans la partie hors-champ qui correspond à une direction allant du champ de vision de l'endoscope à l'instrument chirurgical, pour indiquer la position de l'instrument chirurgical par rapport au champ de vision de l'endoscope.

6. Système chirurgical selon la revendication 4, dans lequel
les informations de chacun desdits un ou plusieurs des premier, deuxième, troisième instruments chirurgicaux qui se situent dans le champ de vision de l'endoscope sont affichées en une position située dans la partie dans-le-champ de la zone d'affichage de position d'instrument chirurgical correspondant à une position d'une extrémité distale de l'instrument chirurgical située dans le champ de vision de l'endoscope.

7. Système chirurgical selon l'une quelconque des revendications 1 à 6, dans lequel
l'interface utilisateur graphique comporte la zone d'affichage de position d'instrument chirurgical à proximité d'une ligne des première, deuxième et troisième zones qui sont agencées en ligne.

8. Système chirurgical selon l'une quelconque des revendications 1 à 7, dans lequel
la première zone affiche une première information d'identification indiquant le premier instrument chirurgical,
la deuxième zone affiche une deuxième information d'identification indiquant le troisième instrument chirurgical,
la troisième zone affiche une troisième information d'identification indiquant le deuxième instrument chirurgical, et
la zone d'affichage de position d'instrument chirurgical affiche les première, deuxième et troisième informations d'identification de manière à indiquer une position des premier, deuxième et troisième instruments chirurgicaux par rapport au champ de vision de l'endoscope.

9. Système chirurgical selon la revendication 8, dans lequel
les première, deuxième et troisième informations d'identification comportent des chiffres, des lettres ou des symboles qui sont différents les uns des autres.

10. Système chirurgical selon l'une quelconque des revendications 1 à 9, dans lequel
dans l'interface utilisateur graphique, les première, deuxième et troisième zones sont agencées en ligne de droite à gauche, et
l'interface utilisateur graphique comporte en outre une quatrième zone agencée sur un côté gauche de la troisième zone, dans lequel la quatrième zone affiche des informations sur l'endoscope.

11. Système chirurgical selon l'une quelconque des revendications 1 à 10, comprenant en outre
une table d'opération (300) pour un patient placée dans un rayon d'actionnement de l'appareil côté patient.

12. Système chirurgical (400) selon l'une quelconque des revendications 1 à 11, dans lequel
l'appareil (100) de commande à distance comporte :
une première poignée d'actionnement (1a) pour que la main droite actionne le premier instrument chirurgical ; et
une deuxième poignée d'actionnement (1b) pour que la main gauche actionne le deuxième instrument chirurgical.

13. Système chirurgical selon la revendication 10, dans lequel l'appareil (100) de commande à distance comprend en outre :
une section (2) à pédales d'actionnement comportant : une première pédale (21b, 22b) configurée pour exécuter une fonction se rapportant au premier instrument chirurgical ; une deuxième pédale (21a, 22a) configurée pour exécuter une fonction se rapportant au deuxième instrument chirurgical ; une troisième pédale (24) configurée pour exécuter une fonction se rapportant à l'endoscope ; et une quatrième pédale (23) configurée pour exécuter une fonction d'embrayage, dans lequel
l'interface utilisateur graphique comporte en outre une cinquième zone agencée sur un côté gauche de la quatrième zone, dans lequel la cinquième zone affiche des informations sur la fonction d'embrayage, de préférence dans lequel
les première, troisième, quatrième et cinquième zones dans l'interface utilisateur graphique sont configurées pour afficher des états d'actionnement des première, deuxième, troisième, quatrième pédales, respectivement.

14. Système chirurgical selon l'une quelconque des revendications 1 à 12, dans lequel l'appareil (100) de commande à distance comprend en outre :
une section à pédales d'actionnement comportant : une première pédale (21b, 22b) configurée pour exécuter une fonction se rapportant au premier instrument chirurgical ; une deuxième pédale (21a, 22a) configurée pour exécuter une fonction se rapportant au deuxième instrument chirurgical ; une troisième pédale (24) configurée pour exécuter une fonction se rapportant à l'endoscope ; et une quatrième pédale (25) configurée pour changer l'un parmi le premier instrument chirurgical à actionner par la première poignée d'actionnement et le deuxième instrument chirurgical à actionner par la deuxième poignée d'actionnement vers le troisième instrument chirurgical.

15. Système chirurgical selon l'une quelconque des revendications 1 à 14, dans lequel l'appareil de commande à distance comprend en outre :
une partie (51a) formant commutateur d'affichage, configurée pour basculer entre le fait de montrer et le fait de cacher la zone d'affichage de position d'instrument chirurgical.
